# EUROPÄISCHE PATENTSCHRIFT

(11) **EP 0 503 162 B1**
(45) Veröffentlichungstag und Bekanntmachung des Hinweises auf die Patenterteilung: **22.04.1998**
(21) Anmeldenummer: 91122406.1
(22) Anmeldetag: 01.01.1992
(51) Int. Cl.: C07D 233/90, A61K 31/415, C07D 233/68, C07D 233/70, C07D 453/02, C07D 403/12, C07D 401/12, C07D 417/12, C07D 405/12, C07D 409/12

(54) **Azol-Derivate, Verfahren zu ihrer Herstellung und ihre Verwendung**
Azole derivatives, process for their preparation and their application
Dérivés d'azole, procédé de leur fabrication et leur application

(30) Priorität: 04.01.1991 DE 4100109; 26.03.1991 DE 4109949; 27.06.1991 DE 4121229
(43) Veröffentlichungstag der Anmeldung: 16.09.1992
(73) Patentinhaber: HOECHST AKTIENGESELLSCHAFT, 65926 Frankfurt am Main (DE)
(72) Erfinder: Wagner, Adalbert, Dr., W-6238 Hofheim am Taunus (DE); Englert, Heinrich, Dr., W-6238 Hofheim am Taunus (DE); Kleemann, Heinz-Werner, Dr., W-6380 Bad Homburg (DE); Gerhards, Hermann, Dr., W-6388 Hofheim am Taunus (DE); Schölkens, Bernward, Prof. Dr., W-6233 Kelkheim/Taunus (DE); Becker, Reinhard, Dr., W-6200 Wiesbaden (DE); Linz, Wolfgang, Dr., W-6500 Mainz 42 (DE); Caille, Jean-Claude, F-49000 Angers (FR); Vevert, Jean-Paul, F-93500 Pantin (FR)

(56) Entgegenhaltungen:
- EP-A- 0 028 834
- EP-A- 0 253 310
- EP-A- 0 323 841
- EP-A- 0 324 377
- EP-A- 0 401 030
- EP-A- 0 409 332
- DE-A- 4 010 797

## Beschreibung

Der Entwicklung neuer Angiotensin-II-Rezeptor-Antagonisten wird wachsende Bedeutung beigemessen. Aus EP-A-28834 sind 1-Benzyl-substituierte Imidazolderivate, aus EP-A-253 310 und EP-A-0 401 030 sind Imidazolderivate mit einer Diarylcarbonsäurefunktion, aus EP-A-323 841 sind Pyrazol- und Triazol-Derivate und aus EP-A-0 409 332 sind Triazol-Derivate jeweils mit Diarylcarbonsäurefunktion sowie aus EP-A-324 377 sind Imidazolderivate mit einer Diaryl-tetrazolyl-gruppe und deren Verwendung als Antagonisten von Angiotensin-II-Rezeptoren bekannt.

Darüber hinaus werden in DE-A 4010797 (entspr. US-Patentanmeldung Nr. 07/679.233) substituierte Azole, die eine Sulfonylharnstoffgruppe enthalten, vorgestellt.

Es wurden neue Azol-Derivate gefunden, die eine neue Sulfonylharnstoff-, Sulfonylurethan- oder eine Sulfonylsulfonamid-Struktur aufweisen und die hochwirksame Antagonisten von Angiotensin-II-Rezeptoren sowohl in vitro als in vivo sind.

Die Erfindung betrifft Verbindungen der Formel (I), 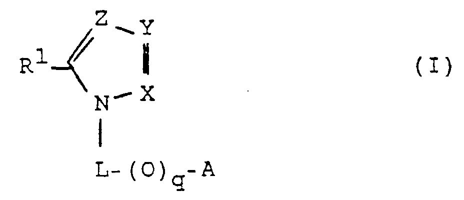 in welcher die Symbole folgende Bedeutungen haben:
- Z: Stickstoff
- X,Y: unabhängig voneinander CR²
- R¹: (C₂-C₇)-Alkyl, (C₃-C₇)-Alkenyl oder (C₃-C₇)-Alkinyl,
- R²: Wasserstoff, Halogen, Nitro, (C₁-C₃)-Perfluoralkyl, Cyano, (C₁-C₁₀)-Alkyl, (C₃-C₁₀)-Alkenyl, -CH₂OR⁵, S(O)ᵣ-R¹⁹, -CO-R⁸ oder -O-R⁶
- R³: 1. Wasserstoff,
2. (C₁-C₈)-Alkyl,
3. (C₃-C₈)-Cycloalkyl,
4. Phenyl,
5. Benzyl oder
6. den unter 2. definierten Rest, worin 1 bis alle H-Atome durch Fluor substituiert sind;
- R⁵: Wasserstoff oder (C₁-C₆)-Alkyl
- R⁶, R⁹: 1. Wasserstoff,
2. (C₁-C₆)-Alkyl, das mit 1-3 Resten aus der Reihe (C₁-C₆)-Alkoxy, das seinerseits mit 1-3 Resten aus der Reihe Hydroxy, (C₁-C₆)-Alkoxy, Amino, Mono-(C₁-C₆)-Alkylamino, Di-(C₁-C₆)-Alkylamino substituiert sein kann, (C₂-C₁₀)-Alkenyl, Hydroxy, Amino, Mono-(C₁-C₆)Alkylamino, Di-(C₁-C₆)-Alkylamino, (C1-C₆)Alkoxycarbonylamino, (C₆-C₁₂)Aryl-(C₁-C₄)Alkoxycarbonylamino; (C₆-C₁₀)-Aryl, (C₆-C₁₀)Aryl- (C₁-C₃)-Alkyl, (C₁-C₉)-Heteroaryl, Carboxy und (C₁-C₄)-Alkoxycarbonyl substituiert sein kann;
3. (C₃-C₈)-Cycloalkyl,
4. (C₃-C₆)-Cycloalkyl-(C₁-₃)-alkyl,
5. (C₆-C₁₂)-Aryl, vorzugsweise Phenyl,
6. (C₆-C₁₀)-Aryl-(C₁-C₄)-Alkyl,
7. (C₁-C₉)-Heteroaryl, welches teilweise oder vollständig hydriert sein kann,
8. (C₁-C₉)- Heteroaryl- (C₁-C₇)-alkyl, wobei der Heteroarylteil teilweise oder vollständig hydriert sein kann,
9. einen wie vorstehend unter 5., 6., 7., und 8. definierten Rest, substituiert mit 1 oder 2 gleichen oder verschiedenen Resten aus der Reihe Halogen, Hydroxy, (C₁-C₄)-Alkyl, Methoxy, Nitro, Cyano, CO₂R³, Trifluormethyl, -NR¹¹R¹² und 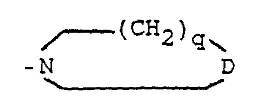
10. (C₁-C₆)-Alkyl, worin 1 bis alle H-Atome durch Fluor substituiert sind,
11. (C₂-C₆)-Alkenyl oder (C₃-C₆)-Alkenoyl,
12. (C₃-C₈)-Cycloalkenyl,
13. (C₃-C₈)-Cycloalkenyl-(C₁-C₃)-Alkyl,
14. (C₆-C₁₀)-Aryl-(C₁-C₄)-Alkyl,
15. (C₆-C₁₀)-Aryl-(C₃-C₆)-Alkenyl,
16. (C₁-C₉)-Hetaryl-(C₃-C₆)-Alkenyl,
17. (C₃-C₆)-Alkinyl,
18. (C₆-C₁₀)-Aryl-(C₃-C₆)-Alkinyl,
19. (C₁-C₉)-Hetaryl-(C₃-C₆)-Alkinyl,
20. R⁶, R⁹ gemeinsam mit dem sie tragenden N-Atom ein Hetaryl, das auch teilweise oder vollständig hydriert sein kann,
- R⁷: Wasserstoff
- R⁸: Wasserstoff oder -OR⁶
- R¹¹,R¹²: unabhängig voneinander Wasserstoff oder (C₁-C₄)-Alkyl
- D: -NR¹³, -O oder -CH₂
- R¹³: Wasserstoff oder (C₁-C₄)-Alkyl
- A: Biphenylrest der mit einem Rest R¹⁵ oder mit R¹⁴ und R¹⁵ zusammen substituiert ist
- R¹⁵: -SO₂-NR⁷-CO-NR⁶R⁹, -SO₂-NH-COO-R⁶, -SO₂-NH-SO₂-NR⁶R⁹, -SO₂-NH-CO-R⁶ oder -SO₂-NH-SO₂-R⁶; oder
- R¹⁴ und R¹⁵: zusammen -CO-NH-SO₂- sein kann
- L: -CH₂-
- g: Null und
- r: Null, 1 oder 2,
sowie deren physiologisch verträgliche Salze.

Alkyl, Alkenyl und Alkinyl können geradkettig oder verzweigt sein. Entsprechendes gilt für davon abgeleitete Reste, wie Alkanoyl oder Alkoxy.

Unter Cycloalkyl werden auch alkylsubstituierte Ringe verstanden.

(C₆-C₁₂)-Aryl ist beispielsweise Phenyl, Naphthyl oder Biphenylyl vorzugsweise Phenyl. Entsprechendes gilt für davon abgeleitete Reste, wie Aroyl oder Aralkyl.

Unter (C₁-C₉)-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Gegebenenfalls vorkommende Stereozentren können sowohl (R)-als auch (S)-konfiguriert sein.

Die Verknüpfung von A erfolgt über eine Alkandiyl-Brücke L, welche vorzugsweise eine Methylengruppe ist.

Die Methylengruppe ist vorzugsweie direkt an den Biphenylrest gebunden.

Unter physiologisch verträglichen Salzen von Verbindungen der Formel (I) versteht man sowohl deren organische als auch anorganische Salze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basiche Gruppen sind unter anderen Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Die Erfindung betrifft auch ein Verfahren zur Herstellung von Verbindungen der Formel (I), sowie von deren physiologisch verträglichen Salzen, das dadurch gekennzeichnet ist, daß man Verbindungen der Formel (II) 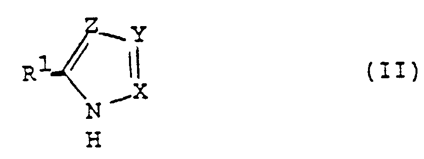 worin R¹, X, Y und Z wie oben definiert sind, alkyliert mit Verbindungen der Formel (III),

U-L-(O)_{q}-A (III)

worin L, A und q wie oben definiert sind, und U für eine Fluchtgruppe steht, gegebenenfalls temporär eingeführte Schutzgruppen wieder abspaltet, erhaltene Sulfonamide der Formel I gegebenenfalls in Urethane der Formel I überführt, erhaltene Sulfonamide der Formel I oder erhaltene Urethane der Formel I gegebenenfalls in Sulfonylharnstoffe der Formel I überführt und die erhaltenen Verbindungen der Formel (I) gegebenenfalls in ihre physiologisch verträglichen Salze überführt.

Geeignete Fluchtgruppen U sind vorzugsweise nucleofuge Gruppen (vgl. Angew. Chem. 72 [1960] 71) wie Halogen, o-Toluolsulfonat, Mesylat oder Triflat.

Verfahren zur Herstellung der Vorstufen der Formel (II) sind u.a. bekannt aus US 4 355 044, EP-A-324 377 und EP-A-323 841.

Weitere Verfahren werden beschrieben von G. L'abbe (Chem. Rev. 69, 345 (1969)), T. Srodsky ("The Chemistry of the Azido Group", Wiley, New York, 1971, S. 331), H. Wamhoff ("Comprehensive Heterocyclic Chemistry) sowie von S. Katritzky Ed., Pergamon Press, New York (1984)).

Ein weiteres Verfahren zur Herstellung von Verbindungen der Formel II geht von 1-Cyanoglyoxylsäure-2-oxim-Derivaten aus und liefert nach Reduktion des Oxims mit literaturbekannten Reduktionsmitteln und Addition von Mercaptoverbindungen an die Nitrilgruppe unter Verwendung geeigneter Schutzgruppen Vorstufen, die unter Wasser-abspaltenden Bedinguhgen zu Imidazolen cyclisiert werden können. Für den Cyclisierungsschritt können u.a. Gemische aus PCl₅ und Dimethylaminopyridin (DMAP), POCl₃ und SOCl₂ und deren Gemische mit DMAP verwendet werden.

Die Oxidation der Thioverbindungen der Formel I mit R² gleich -S(O)ᵣR¹⁹, wobei r gleich Null bzw. 1 ist, zu den entsprechenden Sulfonen und Sulfoxiden erfolgt bevorzugt durch Persäuren in geeigneten Lösungsmitteln wie z.B. Dichlormethan.

Zur Alkylierung der Azole der Formel (II) sind z.B. entsprechnede Benzylhalogenide, -tosylate, -mesylate oder Triflate oder ensprechende Alkylhalogenide, -tosylate, mesylate oder-triflate geeignet.

Die Alkylierung erfolgt nach prinzipiell bekannten Verfahren in analoger Weise.

Azol-Derivat der Formel (II) werden z.B. in Gegenwart einer Base metalliert. Bevorzugte Basen sind Metallhydride der Formel MH wie z.B. Lithium-, Natrium- oder Kaliumhydrid in beispielsweise DMF oder DMSO als Lösungsmittel oder Metallalkoxide der Formel MOR, wobei R für Methyl, Ethyl, t-Butyl steht, und die Reaktion in dem entsprechenden Alkohol, DMF oder DMSO durchgeführt wird. Die so gebildeten Salze der Azole werden in einem aprotischen Lösungsmittel wie DMF oder DMSO gelöst und mit einer geeigneten Menge Alkylierungsreagenz versetzt.

Eine alternative Möglichkeit zur Deprotonierung der Azol-Derivate stellt z.B. die Umsetzung mit Kaliumcarbonat in DMF oder DMSO dar.

Die Umsetzungen werden bei Temperaturen unterhalb Raumtemperatur bis zum Siedepunkt des Reaktionsgemisches, vorzugsweise zwischen +20 °C und dem Siedepunkt des Reaktionsgemisches während etwa 1 bis 10 Stunden durchgeführt.

Die Biphenylderivate können z.B. ausgehend von Arylboronsäurederivaten durch Kopplung mit substituierten Arylhalogeniden mit Übergangsmetallkatalysatoren, insbesondere Palladium synthetisiert werden. Entsprechende Reaktionen werden von R.B. Miller et al. (Organometallics 1984, 3, 1261) oder von A. Zuzuki et al. (Synthetic Commun. 11 (7), 513 (1981)) beschrieben.

Die Sulfonylurethane der Formel I können aus entsprechenden Sulfonamiden der Formel I durch Umsetzung mit Chlorkohlensäureestern in inerten hochsiedenden Lösungsmitteln wie z.B. Toluol bei Temperaturen von ca. 100°C bzw. den Siedepunkten der entsprechenden Lösungsmitteln erhalten werden.

Analog können Sulfonyl-sulfonamide aus den entsprechenden Sulfonamiden durch Umsetzung mit Sulfonsäurechloriden oder Sulfamoylchloriden hergestellt werden.

Der Sulfonamid-Rest kann, falls erforderlich, ausgehend von einer Aminogruppe mittels Meerwein-Reaktion hergestellt werden. Hierfür wird das Hydrochlorid des Amins zuerst diazotiert und dann in Gegenwart eines Kupferkatalysators mit Schwefeldioxid in Eisessig umgesetzt. Nachfolgende Einwirkung von Ammoniak führt zur Sulfonamidogruppe.

Alternativ kann ein entsprechendes Thiophenol durch Oxidation mit Chlor und anschließender Einwirkung von Ammoniak in ein Sulfonamid umgewandelt werden.

Die erfindungsgemäßen Verbindungen der Formel (I) haben antagonistische Wirkung auf Angiotensin-II-Rezeptoren und können deshalb zur Behandlung der Angiotensin-II-abhängige Hypertension verwendet werden. Anwendungsmöglichkeiten bestehen weiterhin bei Herzinsuffizienz, Kardioprotektion, Myocardinfarkt, Herz-Hypertrophie, Arteriosklerosis, Nephropathie, Nierenversagen sowie vasculären Erkrankungen des Gehirn wie transitorischen ischämischen Attacken und Gehirnschlag.

Renin ist ein proteolytisches Enzym aus der Klasse der Aspartyl-Proteasen, welches als Folge verschiedener Stimuli (Volumendepletion, Natriummangel, β-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt.

Postrezeptor-Wirkungen sind unter anderen Stimulation des Phosphoinositol-Umsatzes (Ca²⁺-Freisetzung), Aktivierung der Proteinkinase C) und Facilitation von c-AMP abhängigen Hormon-Rezeptoren.

Die Affinität der Verbindungen der Formel I zum Angiotensin-II-Rezeptor kann durch Messung der ¹²⁵J-Angiotensin-II- oder ³H-Angiotensin-II-Verdrängung von Rezeptoren an Membranen der zona glomerulosa von Rindernebennieren bestimmt werden. Dazu werden die präparierten Membranen in Puffer bei pH 7.4 suspendiert.

Um die Degradierung des Radioliganden während der Inkubierung zu verhindern, wird Aprotinin, ein Peptidase Inhibitor, zugesetzt. Zusätzlich werden ca. 14000 cpm eines Tracers mit spezifischer Aktivität von 74 TBq/mmol (käuflich bei Amersham Buchler, Braunschweig, BRD) und eine Menge Rezeptorprotein, die 50 % des Tracers bindet, verwendet. Die Reaktion wird durch Zugabe von 50 µl Membransuspension zu einer Mischung von 100 µl Puffer + Aprotinin; 50 µl Puffer mit oder ohne Angiotensin-II oder Rezeptorantagonist und 50 µl Tracer gestartet. Nach einer Inkubationszeit von 60 Minuten bei einer Temperatur von 25 °C werden gebundener und freier Radioligand durch einen Filtrationsassay mit Whatmann® GFIC Filtern auf einem Skatron®-Zellsammler getrennt.

Unspezifische Bindungen werden durch Behandlung der Filter mit 0,3 % Polyethylenimin pH=10 verhindert (Sigma, Nr. 3143). Durch Messung der Radioaktivität in einem Gamma-Szintillationszähler wird die Stärke der Verdrängung des Radioliganden vom Rezeptor bestimmt. Die IC₅₀-Werte, welche die Konzentration des Inhibitors bedeuten, um 50 % des Liganden zu verdrängen, werden nach J. Theor. Biol. 59, 253 (1970) bestimmt. Sie liegen für die Verbindungen der Formel (I) im Bereich von 1.10⁻⁴-1.10⁻⁹M.

Alternativ kann die Affinität der Verbindungen der Formel I zum Angiotensin-II-Rezeptor durch Messung der ¹²⁵J-Angiotensin-II- oder ³H-Angiotensin-II-Verdrängung von Rezeptorpräparationen aus verschiedenen Organen (Leber, Lunge, Nebenniere, Hirn etc.) bestimmt werden.

Dazu weden die präparierten Membranen in einem Inkubationspuffer (20 mM Tris, pH 7.4, enthaltend 135 mM NaCl, 10 mM KCl, 10 mM MgCl₂, 5 mM Glucose, 0.2 % Rinderserumalbumin sowie die Proteaseinhibitoren PMSF 0,3 mM und Bacitracin 0,1 mM) suspendiert und zusammen mit dem radioaktiv markierten Angiotensin-II und verschiedenen Konzentrationen der zu testenden Verbindungen für 90 min bei 25°C inkubiert. Anschließend werden gebundener und freier Radioligand durch Filtration über Microglasfaserfilter (GF51, Schleicher & Schüll) auf einem Zellsammler (SKATRON) getrennt.

Durch Messung der Rezeptor-gebundenen Radioaktivität auf den Filtern mittels eines Beta- oder Gamma-Spektrometers wird der Grad der Verdrängung des Radioliganden vom Rezeptor durch die Testverbindungen bestimmt. Die Stärke der Verdrängung des Radioliganden vom Rezeptor durch die Testverbindungen wird mit der IC₅₀ angegeben, d.i. die Konzentration des Inhibitors, die 50 % des gebundenen Radioliganden vom Rezeptor verdrängt. Die Berechnung der IC₅₀ Werte erfolgt mittels einer PC-Software (LIGAND, G.A. McPherson 1985, Elsevier-BIOSOFT, 68 Hills Road, Cambridge CB 2 1LA, UK.). Die für Verbindungen der Formel (I) gemessenen IC₅₀ Werte liegen im Bereich von 1 x 10⁻⁵ bis 1 x 10⁻¹¹ M (vergl. nachfolgende Tabelle 1,in der die IC₅₀-Werte für erfindungsgemäße Verbindungen zusammengestellt sind).

**Tabelle 1**

| Beispiel | IC₅₀ [nM] |
|---|---|
| 1 | 5000 |
| 2 | 8000 |
| 3 | 1100 |
| 4 | 1100 |
| 5 | 16000 |
| 22 | 2000 |
| 24 | 800 |
| 25 | 1400 |
| 29 | 1,1 |
| 30 | 2030,0 |
| 31 | 153,0 |
| 32 | 3,5 |
| 33 | 34,0 |
| 34 | 1,0 |
| 35 | 50,0 |
| 36 | 16,0 |
| 37 | 1,1 |
| 55 | 8,8 |
| 56 | 4,6 |
| 57 | 1100 |
| 58 | 3,0 |
| 59 | 1,3 |
| 60 | 2,2 |
| 61 | 1,1 |
| 62 | 3,6 |
| 63 | 1,3 |
| 64 | 0,5 |
| 65 | 1,8 |
| 66 | 6,9 |
| 67 | 0,91 |
| 68 | 12,0 |
| 69 | 3,2 |
| 70 | 4,4 |
| 71 | 2,2 |
| 73 | 2,5 |
| 76 | 9,5 |
| 79 | 5,8 |
| 80 | 0,69 |
| 81 | 0,79 |
| 83 | 0,96 |
| 84 | 4,3 |
| 85 | 3,9 |
| 89 | 1,1 |
| 90 | 0,69 |
| 92 | 280,0 |
| 93 | 3,3 |
| 95 | 1,8 |
| 98 | 1,4 |
| 99 | 26,6 |
| 100 | 68,5 |
| 101 | 2,4 |
| 102 | 2,3 |
| 105 | 3,0 |
| 107 | 2,5 |
| 108 | 0,95 |
| 109 | 0,6 |
| 110 | 0,5 |
| 111 | 2,9 |
| 112 | 1,5 |
| 113 | 0,3 |
| 115 | 0,9 |
| 116 | 2,4 |
| 117 | 1,2 |
| 124 | 1,8 |
| 125 | 2,8 |
| 127 | 3,0 |
| 128 | 5,6 |
| 129 | 1,5 |
| 134 | 180,0 |
| 135 | 5,6 |
| 138 | 1,7 |
| 139 | 2,8 |
| 140 | 8,2 |
| 141 | 4,4 |
| 144 | 5,3 |
| 146 | 40,0 |
| 151 | 0,4 |
| 152 | 1,5 |
| 153 | 0,88 |
| 154 | 1,8 |
| 155 | 6,0 |
| 156 | 4,7 |
| 157 | 1,4 |
| 159 | 8,7 |
| 160 | 0,73 |
| 161 | 57,0 |
| 162 | 3,9 |
| 163 | 3,7 |
| 164 | 0,86 |
| 165 | 2,3 |
| 166 | 1,2 |
| 167 | 4,0 |
| 168 | 7,0 |
| 169 | 2,9 |
| 170 | 2,7 |
| 171 | 0,7 |
| 172 | 0,48 |
| 174 | 5,1 |
| 179 | 2,6 |
| 181 | 1,0 |
| 183 | 1,7 |
| 185 | 5,9 |
| 186 | 6,5 |
| 187 | 1,2 |
| 190 | 22,0 |
| 191 | 21,4 |
| 194 | 21,7 |
| 195 | 3,0 |

Zur Bestimmung der antagonistischen Wirkung der Verbindungen der Formel (I) kann ihr Effekt auf den Angiotensin-II-induzierten Blutdruckanstieg an narkotisierten Sprague-Dawley Ratten gemessen werden. Als Narkotikum dient Na-Thiobarbital (Trapanal®, Warenzeichen von Byk Gulden, BRD) in der Dosierung 100 mg/kg i.p. Die i.v.-Applikation erfolgt in die Vena Jugularis. Der Blutdruck wird in der A. carotis gemessen. Zunächst werden die Tiere mit Pentoliniumtartrat (10 mg/kg i.m.) vorbehandelt, so daß ein niedrigerer Blutdrucklevel erreicht wird (Ganglienblockade). ANG II (Hypertensin CIBA) wird im Volumen 0,1 ml/100 g in 10-minütigen Intervallen i.v. appliziert. Die Dosis beträgt 0,5 µg/kg. Die Verbindungen der Formel (I) werden in Aqua. dest. gelöst und in den Dosierungen 0,1 bis 1,0 mg/kg intravenös bzw. 10 und 100 mg/kg intraduodenal appliziert.

Die Verbindungen der Formel (I) sind insbesondere im Bereich von 0,1-100 mg/kg, bevorzugt 0,1-3 mg/kg, wirksam.

Die Erfindung bezieht sich ebenso auf pharmazeutische Zusammensetzungen bestehend aus einer Verbindung der Formel (I) und anderen Wirkstoffen, wie z.B. Diuretika oder nichtsteroidalen anti-inflammatorischen Wirkstoffen. Die Verbindungen der Formel (I) können auch als Diagnostika für das Renin-Angiotensin-System verwendet werden.

Pharmazeutische Präparate enthalten eine wirksame Menge des Wirkstoffs der Formel (I) und eventuell andere Wirkstoffe zusammen mit einem anorganischen oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, dem Alter und von der Applikationsart ab.

Die pharmazeutischen Präparate der vorliegenden Erfindung werden in an sich bekannten Lösungs-, Misch-, Granulier- oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen wie Tabletten, Dragees, Steckkapseln, wäßrige, alkoholische oder ölige Suspensionen oder wäßrige, alkoholische oder ölige Lösungen gebracht. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung als Trocken- und Feuchtgranulat erfolgen. Als ölige Trägerstoffe oder Lösungsmittel kommen beispielsweise pflanzliche oder tierische Öle, wie Sonnenblumenöl und Lebertran in Betracht.

Zur subkutanen oder intravenösen Applikation werden die aktiven Verbindungen oder deren physiologisch verträgliche Salze, gegebenenfalls mit den dafür üblichen Substanzen wie Lösungsvermittlern, Emulgatoren oder weiterem Hilfsstoffen, in Lösungen, Suspensionen oder Emulsionen gebracht. Als Lösungsmittel kommen z.B. Wasser, physiologische Kochsalzlösung oder Alkohole, wie Ethanol, Propandiol oder Glycerin, sowie Zuckerlösungen wie Glucose- oder Mannitlösungen oder Mischungen aus den genannten Lösungsmitteln in Frage.

Liste der Abkürzungen:
- DMF: N,N-Dimethylformamid
- NBS: N-Bromsuccinimid
- AIBN: α,α-Azobis-isobutyronitril
- EI: electron impact
- DCI: Desorption-Chemical Ionisation
- RT: Raumtemperatur
- EE: Ethylacetat (EtOAc)
- DIP: Diisopropylether
- MTB: Methyltertiärbutylether
- mp: Schmelzpunkt
- HEP: n-Heptan
- DME: Dimethoxyethan
- FAB: Fast Atom Bombardment
- CH₂Cl₂: Dichlormethan

Die Erfindung wird durch die nachfolgenden Beispiele erläutert:

### Beispiel 1

Synthese von 1-[(2'-Phenylaminocarbonyaminosulfonylbiphenyl-4-yl)-methyl]-2-n-butyl-4-chloro-imidazol-5-carboxaldehyd 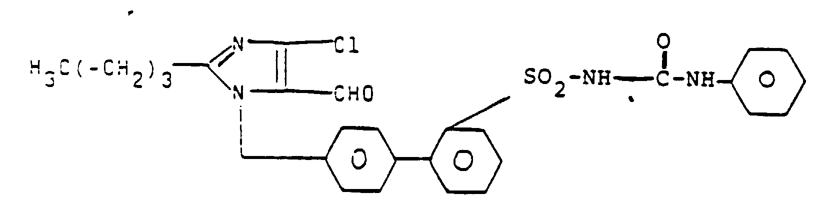
a) Herstellung von 4'-Methylbiphenyl-2-amin
   Zu 23.9 g (0.112 Mol) 4'-Methyl-2-nitrobiphenyl (R.B. Müller und S. Dugar, Organometallics 1984, 3, 1261) in 50 ml Methanol gibt man 3 g Raney-Nickel und hydriert unter Normaldruck bei Raumtemperatur bis zur Aufnahme der theoretischen Menge H₂. Danach wird der Katalysator zur Filtration abgetrennt und das Filtrat eingeengt. Chromatographie an SiO₂ (500 g) mit EE/HEP(1/6) als Laufmittel liefern 19 g der Titelverbindung als Öl (92.5 %)
   R_{f}(EE/HEP 1/4) = 0.3 MS(EI) = 183 (M⁺)
b) 4'-Methyl-biphenyl-2-ammoniumhydrochlorid
   10 g Verbindung 1a) werden in 50 ml 6N HCl und 100 ml Dioxan gelöst. Abdestillieren des Lösungsmittels liefert die Titelverbindung, die ohne weitere Reinigung verwendet wird.
c) 4'-Methylbiphenyl-2-sulfonamid
   Zu einer Suspension von 31 g (140 mmol) Verbindung 1b) in 200 ml 6N HCl gibt man bei -10°C 7.9 g (114 mmol) Natriumnitrit, wobei eine klare Lösung entsteht. Diese wird bei 0°C zu einer Lösung bestehend aus 200 ml Eisessig, gesättigt mit SO₂, 17 g CuCl₂.H₂O und 25 ml H₂O addiert. Man läßt danach auf Raumtemperatur kommen und rührt 2 Stunden bei dieser Temperatur. Nun gibt man 250 ml EE zu, trennt die Phasen und trocknet die organische Phase mit Magnesiumsulfat. Einengen liefert ein Öl, welches in 300 ml Aceton gelöst wird. Anschließend addiert man 150 ml 25 %igen Ammoniak und rührt 2 Stunden. Nun wird eingeengt und 500 ml EE zugegeben. Die EE-Phase wird 1x mit H₂O gewaschen, mit MgSO₄ getrocknet und eingeengt. Chromatographie an SiO₂ mit EE/HEP(1/1) liefert die Titelverbindung (4.6 g).
   R_{f} (EE/HEP 1/1) = 0.25 MS(DCI) = 248 (M⁺+H)
   Schmp.: 122°C
d) 4'-Methylbiphenyl-2-N,N-dimethylaminoformylsulfonamid
   4.6 g (18.6 mmol) Verbindung 1c) und 2.5 g (19.3 mmol) N,N-Dimethylformamiddimethylacetal in 30 ml DMF werden 2 Stunden bei Raumtemperatur gerührt, nun werden 100 ml H₂O zugegeben und der gebildete Niederschlag abgesaugt und an der Luft getrocknet, wobei man 4.2 g der Titelverbindung erhält
   R_{f} (EE/HEP 1/1) = 0.2 MS (DCI) = 303 (M⁺+H)
e) 4'-Brommethylbiphenyl-2-N,N-dimethylamino-formylsulfonamid
   Zu 3.76 g (13.5 mmol) der Verbindung 1d) und 2.4 g NBS (13.5 mmol) in 50 ml Chlorbenzol werden 150 mg Benzoylperoxid gegeben. Nach 4 Stunden unter Rückfluß wird eingeengt, 50 ml EE zugegeben und die EE-Phase 1x mit 10 %iger Na₂SO₃-Lösung und 1x mit H₂O gewaschen. Nach Trocknen mit Na₂SO₄ wird eingeengt und an SiO₂ chromatographiert. (Laufmittel EE/HEP 2/1). Man erhält 1.2 g der Titelverbindung.
   R_{f} (EE/HEP 2/1) = 0.23 MS (DCI) = 381, 383 (M⁺+H)
f) 2-n-Butyl-4-chloro-5-formyl-imidazol
   Zu 20 g (0,106 Mol) 2-n-Butyl-4-chloro-5-hydroxymethylimidazol (hergestellt nach EP-A 253 310) in 350 ml Eisessig gibt man bei 10-15°C langsam 305 ml einer 1 M (NH₄)₂Ce(NO₃)₆-Lösung in H₂O zu. Nach 2,5 h bei Raumtemperatur wird der pH-Wert mit 2 N KOH auf 4 eingestellt (20°C während der Zugabe der Base). Nun wird 4x mit je 500 ml CH₂Cl₂ extrahiert und die vereinigten organischen Extrakte 3x mit je 300 ml gesättigter wäßriger NaHCO₃-Lösung gewaschen, mit Na₂SO₄ getrocknet und eingeengt, wobei die Titelverbindung als farbloser Feststoff anfällt (18 g, 92 %).
   Smp.: = 90°C.
   R_{f} (DIP/MTB 1/1) = 0.5
g) 1-[(2'-N,N-Dimethylaminoformylsulfonamidobipheny1-4-yl)-methyl]-2-n-butyl-4-chloro-imidazol-5-carboxaldehyd
   690 mg (1.98 mmol) Verbindung 1e), 370 mg (1.98 mmol) Verbindung 1f) und 270 mg (1.98 mmol) Kaliumcarbonat werden in DMF (10 ml) bei Raumtemperatur 2 Stunden lang gerührt. Danach werden 50 ml EE zugegeben und 2 mal mit H₂O gewaschen. Die organische Phase wird getrocknet (Na₂SO₄) und eingeengt. Chromatographie an SiO₂ mit EE/HEP (2/1) als Laufmittel liefert die Titelverbindung (380 mg; 40 %)
   R_{f} (EE/HEP 2/1) = 0.15 MS (DCI) = 487 (M⁺+H)
h) 1-[(2 -Sulfonamidobiphenyl-4-yl)-methyl]-2-n-butyl-4-chloro-imidazol-5-carboxaldehyd
   280 mg (0.58 mmol) Verbindung 1 g) in 7 ml Methanol und 14 ml H₂O werden mit 110 mg (2.88 mmol) Natriumhydroxid versetzt und 4 Stunden zum Sieden erhitzt. Nach Abkühlen auf Raumtemperatur wird mit 4 N HCl auf pH etwa 6 eingestellt und 3x mit 30 ml EE extrahiert, die EE-Phasen getrocknet (Na₂SO₄) und eingeengt, wobei man 190 mg der Titelverbindung erhält.
   R_{f} (EE/HEP 2/1) = 0.45 MS (DCI) = 432 (M⁺+H)
i) 1-[(2'-Phenylaminocarbonylaminosulfonylbiphenyl-4-yl)-methyl]-2-n-butyl-4-chloro-imidazol-5-carboxaldehyd
   730 mg (1.69 mmol) Verbindung 1h) werden in 10 ml Phenylisocyanat auf 80°C erhitzt. Nach 4 Stunden wird eingeengt und an SiO₂ chromatographiert (Laufmittel EE/HEP (2/1)), wobei 400 mg der Titelverbindung anfallen.
   R_{f} (EE/HEP 2/1) = 0.15 MS (DCI) = 551 (M⁺+H)

### Alternative Herstellung der Verbindung 1d (4'-Methyl-2-N,N-dimethylaminoformylsulfonamid)

Zu 11 g (37,9 mmol) 2-N,N-Dimethylaminoformylsulfonamidobrombenzol (hergestellt aus 2-Bromanilin analog 1b - 1d); 1 g Triphenylphosphin, 8 g Na₂CO₃ in 150 ml Toluol und 40 ml H₂O gibt man unter Argon zuerst 420 mg Pd(OAc)₂ und anschließend 5,66 g (41.9 mmol) 4-Tolylboronsäure in 100 ml Ethanol. Nun wird 4 h zum Sieden erhitzt. Danach wird eingeengt und in 500 ml Essigsäureethylester und 500 ml H₂O aufgenommen. Der entstehende Niederschlag wird abfiltriert und als Titelverbindung charakterisiert. Die Essigsäureethylesterphase wird abgetrennt, getrocknet (Na₂SO₄) und eingeengt. Chromatographie an SiO₂ mit Essigsäureethylester liefert einen weiteren Anteil an Titelverbindung (insgesamt 7.6 g = 66 %)

### Alternative Herstellung von 2-Brombenzolsulfonamid (der analogen Zwischenstufe Zu 1c)

Zu 4,7 g 2-Bromthiophenol in 60 ml H₂O leitet man bei 0°-10°C für 30 min Cl₂-Gas ein. Danach wird 30 min bei 0°C gerührt und anschließend ohne Kühlung 30 min lang Luft durch die Lösung geblasen. Nach Zugabe von 60 ml Aceton und abermaligem Abkühlen auf 0°C tropft man langsam 10 ml gesättigte NH₄OH-Lösung zu. Nach weiteren 30 min wird der pH-Wert der Lösung mit 4 n HCl auf den Wert 3 eingestellt und das Produkt durch Filtration gewonnen.
Ausbeute 4.5 g (77 %)
Smp. = 190 - 191°C R_{f} (EE/H 1/1) = 0.4

### Beispiel 2

Die Synthese von 1-[(2'-n-Propylaminocarbonylaminosulfonylbiphenyl-4-yl)-methyl]-2-n-butyl-4-chloroimidazol-5-carboxaldehyd erfolgte analog zu Beispiel 1
R_{f} (EE) = 0.6 MS (FAB) = 517 (M⁺+H)

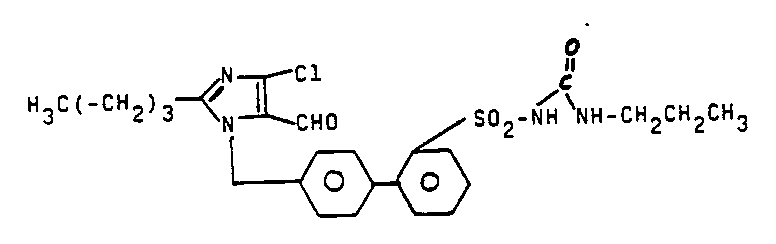

### Beispiel 3

Synthese von 1-[(2-Pyridyl-2-aminocarbonylaminosulfonylbiphenyl-4-yl)methyl]-2-n-butyl-4-chloroimidazol-5-carboxaldehyd 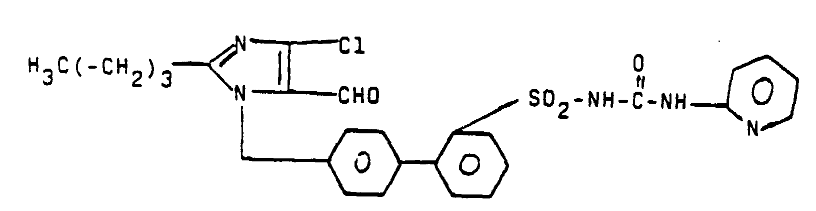
a) 1-[(2'-Ethoxycarbonylaminosulfonylbiphenyl-4-yl)methyl]-2-n-butyl-4-chloro-imidazol-5-carboxaldehyd Zu 1.1 g (2.5 mmol) Verbindung 1h) und 0.78 g (5,6 mmol) Kaliumcarbonat in 20 ml trockenem DME werden 0.48 ml (5.1 mmol) Chlorameisensäureethylester zugesetzt. Nach 1 Stunde läßt man auf Raumtemperatur abkühlen und versetzt mit 50 ml 10 %iger KH₂PO₄-Lösung. Nach Extraktion mit EE wird mit Na₂SO₄ getrocknet und eingeengt. Chromatographie an SiO₂ mit EE/HEP (2/1) als Laufmittel liefert 840 mg Titelverbindung.
   R_{f} (EE/HEP 2/1) = 0.32 MS (DCI) = 504 (M⁺+H)
b) 1-[(2'-Pyridyl-2-aminocarbonylaminosulfonylbiphenyl-4-yl)-methyl]-2-n-butyl-4-chloto-imidazol-5-carboxaldehyd 150 mg (0.3 mmol) Verbindung 3a) und 28,5 mg(0.3 mmol) 2-Aminopyridin werden in 8 ml trockenem Toluol 2 Stunden zum Sieden erhitzt. Anschließend wird eingeengt und an SiO₂ chromatographiert (Laufmittel EE), wobei 34 mg der Titelverbindung anfallen.
   R_{f} (EE/Methanol 10/1) = 0.4 MS (FAB) = 552 (M⁺+1)

Die Verbindungen der Beispiele 4-39 lassen sich analog zu Beispiel 3 synthetisieren.
Diese Verbindungen besitzen folgende allgemeine Formel (A)

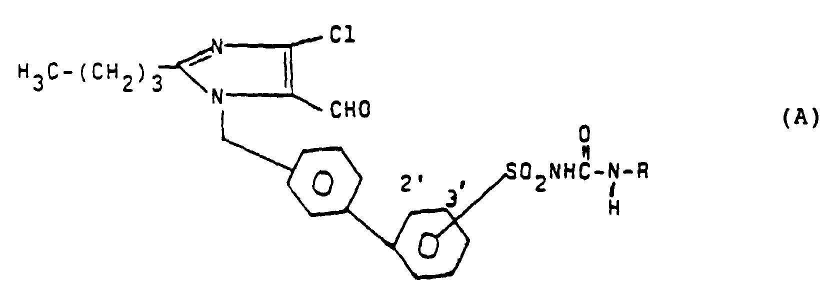

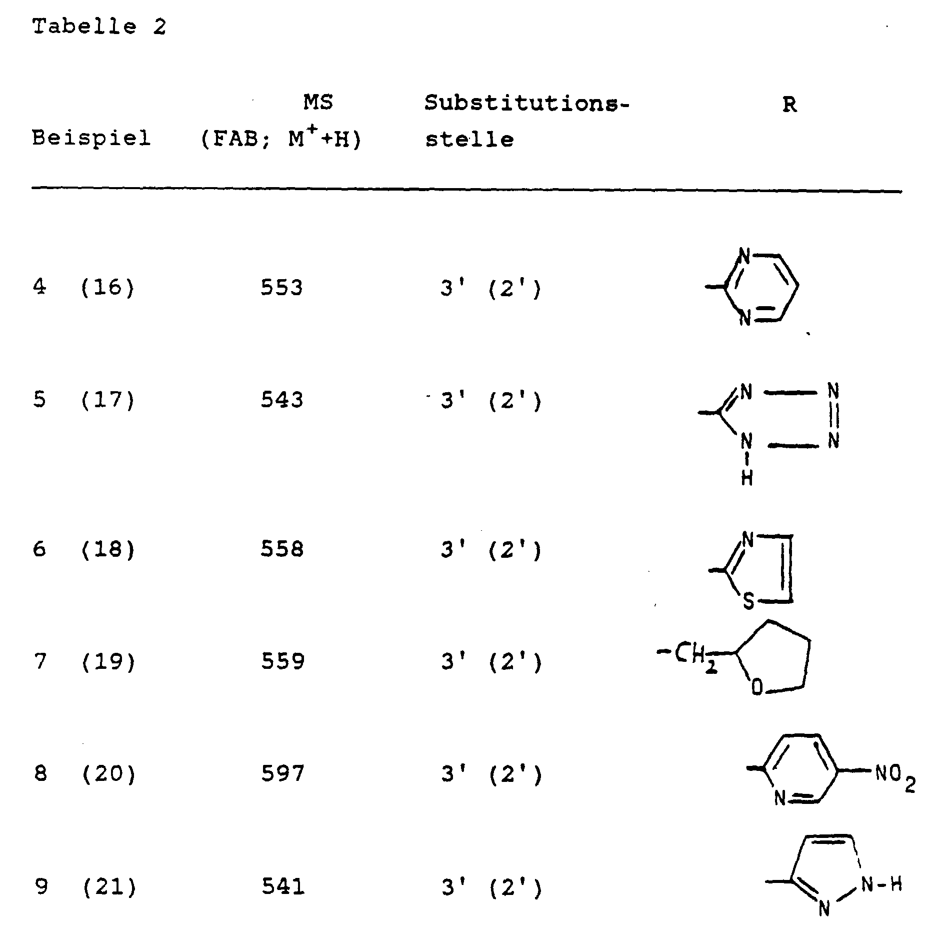

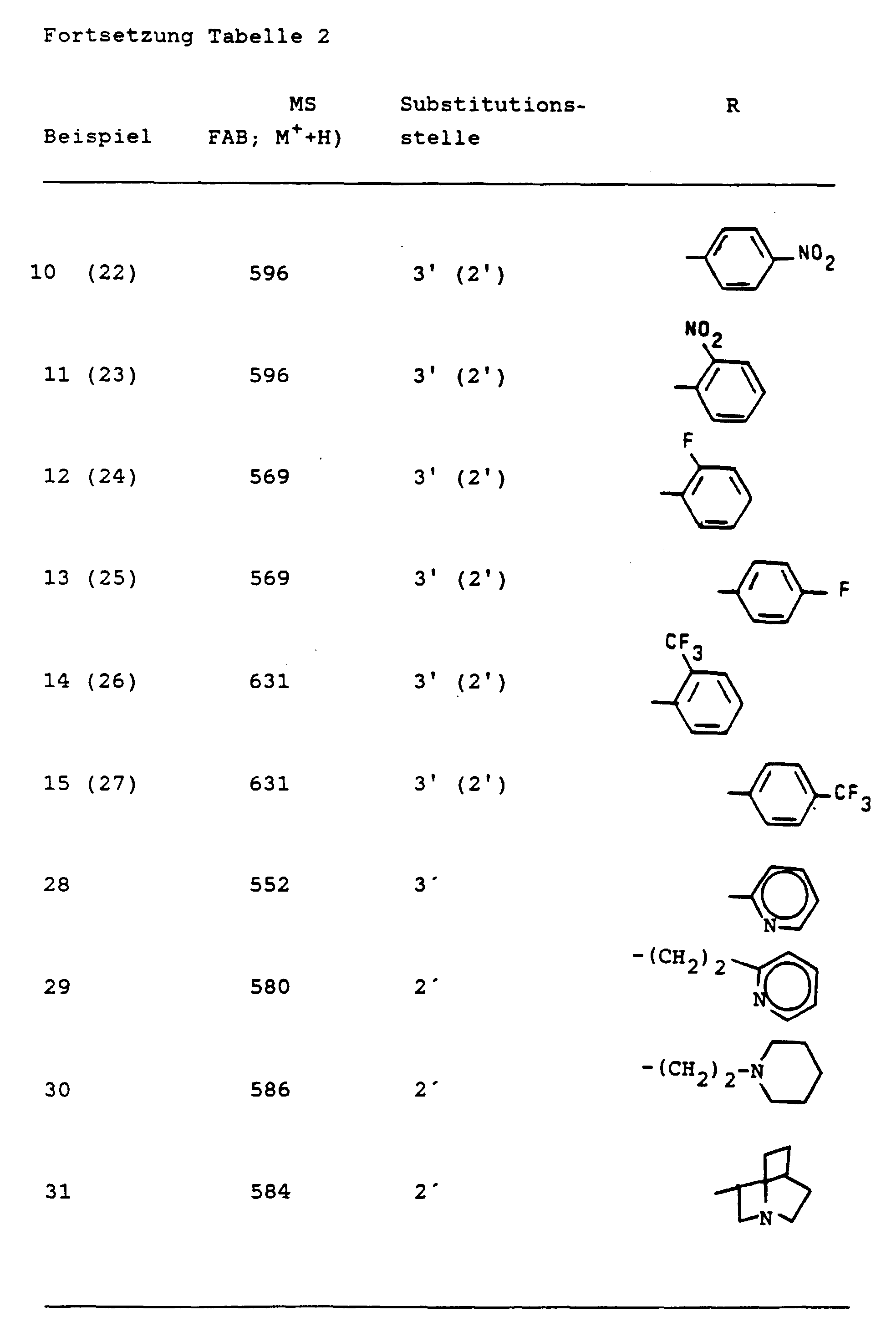

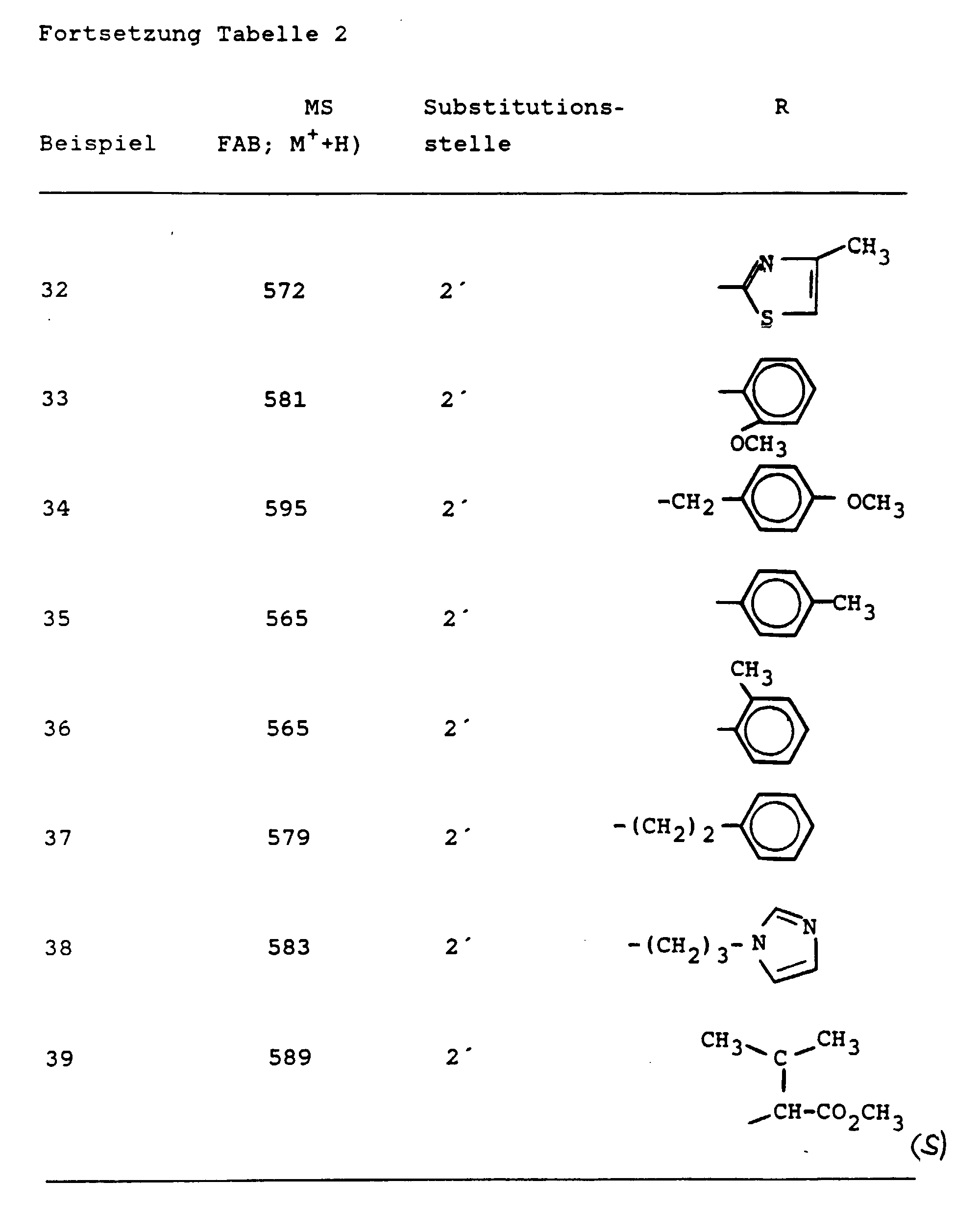

### Beispiel 40

Synthese von 1-[(2'-Phenylaminocarbonylaminosulfonylbiphenyl-4-yl)-methyl]-2-n-butyl-4-chloro-5-hydroxymethylimidazol 100 mg (0.18 mmol) Verbindung 1) werden in 5 ml Ethanol gelöst und bei Raumtemperatur mit 10 mg (0.27 mmol) Natriumborhydrid versetzt. Nach 20 Stunden gibt man 10 ml 5 %ige Natriumhydrogensulfat-Lösung zu und extrahiert 3x mit EE. Die organische Phase wird mit Na₂SO₄ getrocknet und eingeengt. Chromatographie an SiO₂ mit EE/HEP (3/1) liefert 55 mg der Titelverbindung.
R_{f} (EE/HEP 3/1) = 0.25 MS (DCI) = 553 (M⁺+H)

Die Verbindungen (siehe Formel B) der Beispiele 41-54 wurden analog zu Beispiel 40 aus den Verbindungen der Beispiele 2, 3 und 16-27 synthetisiert (Tabelle 3)

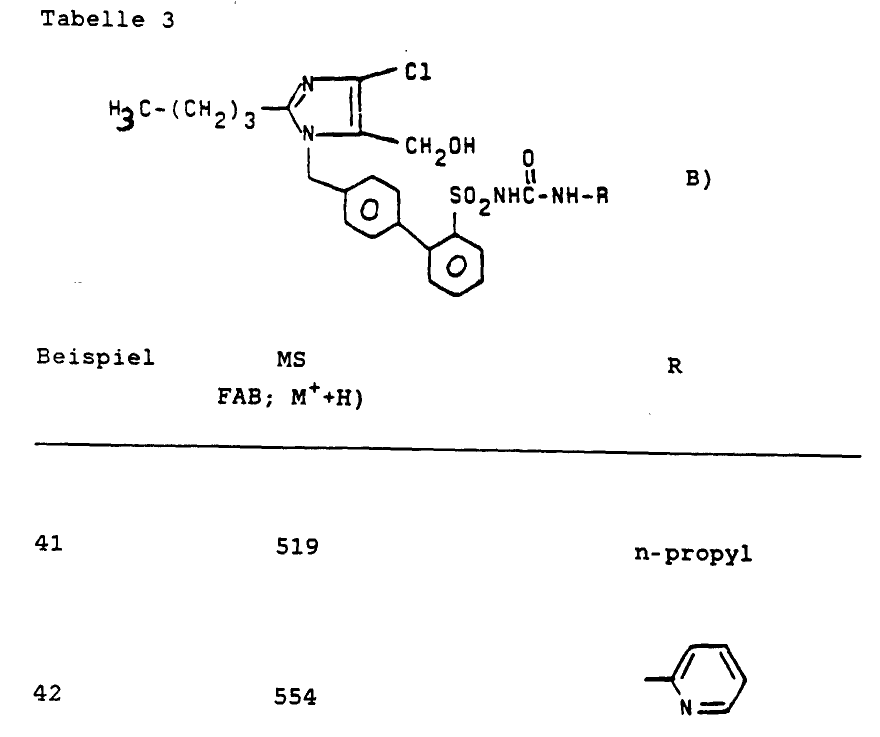

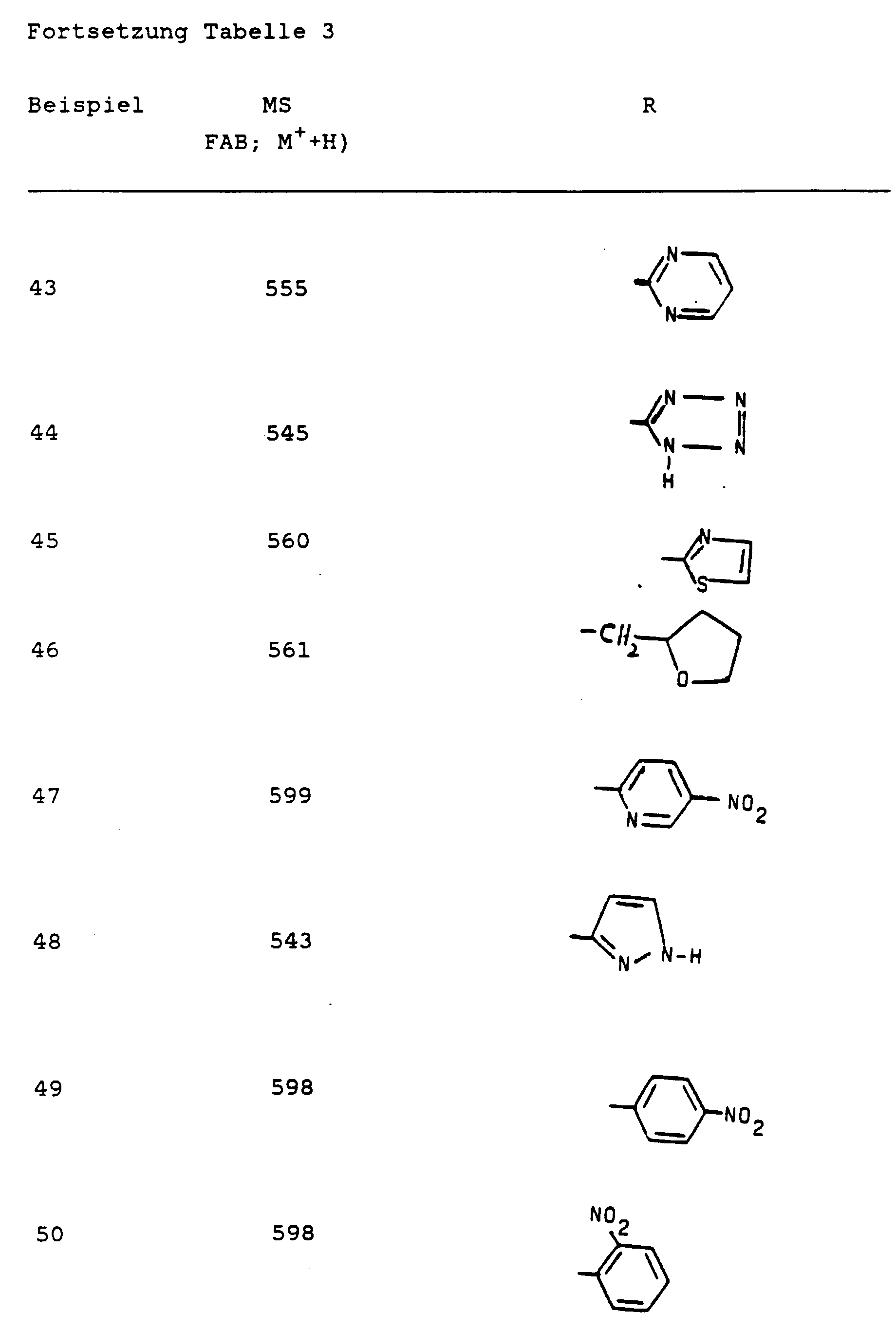

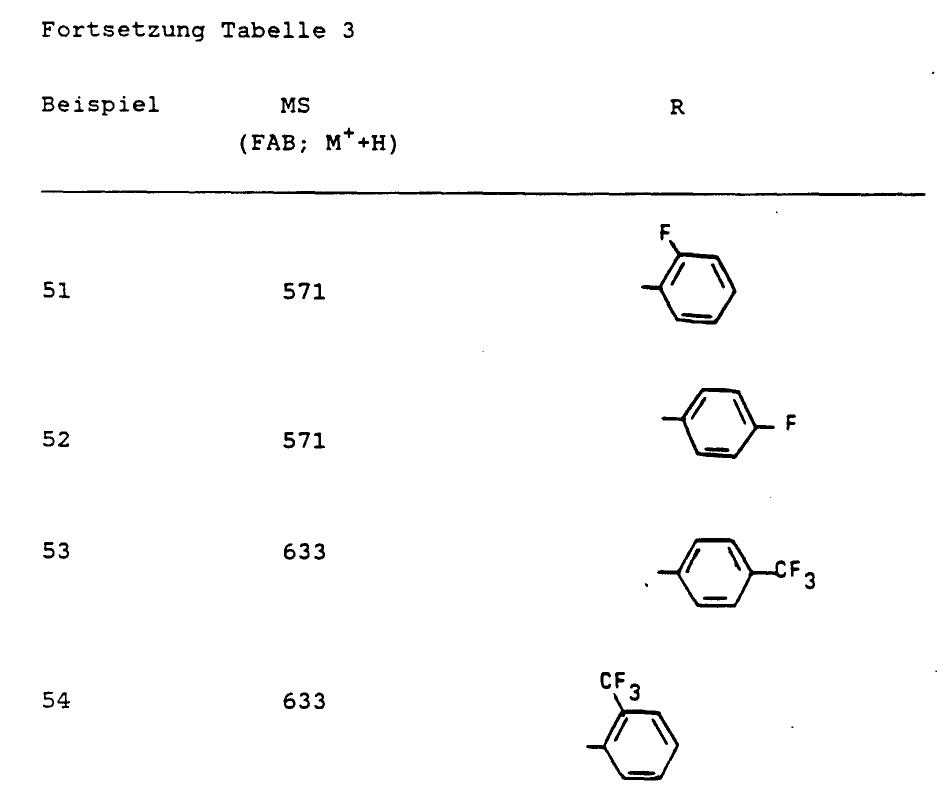

### Beispiel 55

Herstellung von 1-[(2'-Allylaminocarbonylaminosulfonylbiphenyl-4-yl)-methyl]-2-n-butyl-4-chloro-imidazol-5-carboxyaldehyd 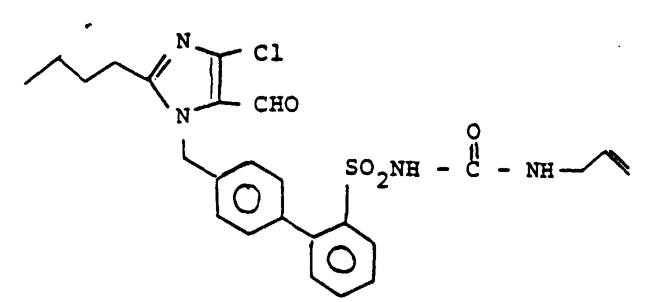 730 mg (1.69 mmol) Verbindung 1 h) werden in 10 ml Allylisocyanat auf 80°C erhitzt. Nach 4 Stunden wird eingeengt und an SiO₂ chromatographiert (Laufmittel EE/HEP (2/1)), wobei 400 mg der Titelverbindung anfallen.
R_{f} (EE/HEP 2/1) = 0.15 MS (FAB) = 515 (M⁺+H)

### Beispiel 56

Herstellung von 1-[(2'-Allylaminocarbonylaminosulfonylbiphenyl-4-yl)-methyl]-2-n-butyl-4-methylthio-imidazol-5-carbonsäure 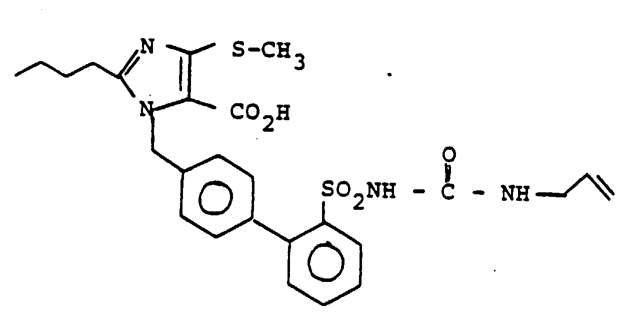
a) 2-Amino-2-cyano-essigsäureethylester
   Zu 35 g (0.246 Mol) 2-Cyanoglyoxylsäureethylester-2-oxim in 350 ml H₂O und 280 ml gesättigter Natriumhydrogencarbonat-Lösung werden bei Raumtemperatur portionsweise (15 min) 119 g Natriumdithionit zugegeben. Anschließend wird 1 Stunde auf 35°C erwärmt; dann mit NaCl gesättigt und 5x mit Dichlormethan extrahiert. Nach Trocknen mit Calciumchlorid wird die organische Phase eingeengt. Man erhält 11.8 g der Titelverbindung als Öl.
   R_{f} (CH₂Cl₂/CH₃OH 9/1) = 0.6
b) 2-Cyano-2-n-butylcarbonylaminoessigsäureethylester
   Zu 3.6 g (28.09 mmol) Verbindung 56a) in 50 ml trockenem CH₂Cl₂ und 2.3 ml (28.09 mmol) Pyridin tropft man bei -5°C bis 0°C 3.39 ml (28.09 mmol) Valeroylchlorid in 5 ml CH₂Cl₂ zu. Anschließend wird 1 Stunde bei Raumtemperatur gerührt. Die organische Phase wird dann 3x mit H₂O und 1x mit gesättigter NaCl-Lösung gewaschen, mit Calciumchlorid getrocknet und eingeengt. Kristallisation aus DIP liefert 1.7 g der Titelverbindung.
   R_{f} (CH₂Cl₂/CH₃OH 9/1) = 0.35
   Schmp.: 87°C
c) 3-Amino-2-n-butylcarbonylamino-methylthioacrylsäureethylester
   Zu 2.9 g (13.67 mmol) Verbindung 56b) und 0.19 ml (1.36 mmol) Triethylamin in 60 ml absolutem Ethanol gibt man bei Raumtemperatur 2 ml (27.26 mmol) kondensiertes Methylmercaptan. Nach 3 Tagen gibt man nochmals 0.5 ml Methylmercaptan zu. Nach weiteren 24 Stunden bei Raumtemperatur werden nochmals 0.5 ml Methylmercaptan und 0.19 ml Triethylamin zugespritzt und weitere 24 Stunden bei Raumtemperatur gerührt. Anschließend wird das Lösungsmittel entfernt und der Rückstand aus DIP kristallisiert, wobei 2.4 g der Titelverbindung anfallen.
   R_{f} (CH₂Cl₂/EE 4/1) = 0.3
   Schmp.: 120°C
d) 2-n-Butyl-4-methylthio-imidazol-5-carbonsäureethylester
   Zu 4.17 g (20.0 mmol) Phosphorpentachlorid in 20 ml CH₂Cl₂ tropft man bei -78°C 2.44 g (20.0 mmol) 4-Dimethylaminopyridin in 12 ml CH₂Cl₂. Nach 5 min werden 2.42 g (10.0 mmol) Verbindung 56c in 25 ml CH₂Cl₂ zugetropft. Nun läßt man auf Raumtemperatur kommen und verdünnt mit 30 ml CH₂Cl₂. Nach 2 Stunden werden unter Eiskühlung 300 ml 1N Natriumhydrogencarbonat-Lösung zugegeben und 1 Stunde gerührt. Nun werden die Phasen getrennt, die wäßrige Phase 3x mit EE extrahiert und die vereinigten organischen Phasen mit Calciumchlorid getrocknet. Chromatographie an SiO₂ mit CH₂Cl₂/EE (9/1)
   R_{f} (CH₂Cl₂/EE 9/1) = 0.6 MS (DCI) = 243 (M⁺+H)
e) 1-[(2'-Sulfonamidobiphenyl-4-yl)-methyl]-2-n-butyl-4-methylthio-imidazol-5-carbonsäure-ethylester
   Zu 1.35 g (2.5 mmol) 1-[(2'-N,N-Dimethylaminoformylsulfonamidobiphenyl-4-yl)-methyl]-2n-butyl-4-methylthio-imidazol-5-carbonsäurethylester [hergestellt aus Beispiel 56d) und Beispiel 1 e) analog Beispiel 1 g)] in 30 ml Methanol werden 15 ml konz. HCl zugegeben. Nach 90 min unter Rückfluß läßt man auf Raumtemperatur abkühlen und stellt mit 2N NaOH-Lösung einen pH=5-6 ein. Nun wird 3x mit je 100 ml EE extrahiert, die organischen Extrakte mit Na₂SO₄ getrocknet und eingeengt, wobei die Titelverbindung als Schaum anfällt, der ohne weitere Reinigung für den nächsten Reaktionsschritt eingesetzt wird.
   R_{f} (EE/HEP 1/1) = 0,2 MS (FAB) = 488 (M+H)
f) Die Titelverbindung 56 erhält man, indem man 120 mg 1-[(2'-Allylaminocarbonylamino-sulfonyl-biphenyl-4-yl)methyl]-2-n-butyl-4-methylthio-imidazol-5-carbonsäureethylester (erhältlich analog Beispiel 55)) in 10 ml Ethanol und 1 ml 2 N Natriumhydroxyd-Lösung 4 Tage bei Raumtemperatur rührt. Danach wird eingeengt, H₂O zugegeben und mit 1 n HCl auf pH = 4 eingestellt, wobei die Titelverbindung ausfällt und durch Filtration isoliert wird.
   R_{f} (EE/MeOH 10/1) = 0.1 MS (FAB) = 543 (M+H)

### Beispiel 57

Synthese von 1-[(2'-Pyridylethyl-2-aminocarbonylaminosulfonylbiphenyl-4-yl)methyl]-2-n-butyl-4-methylthioimidazol-4-carbonsäure 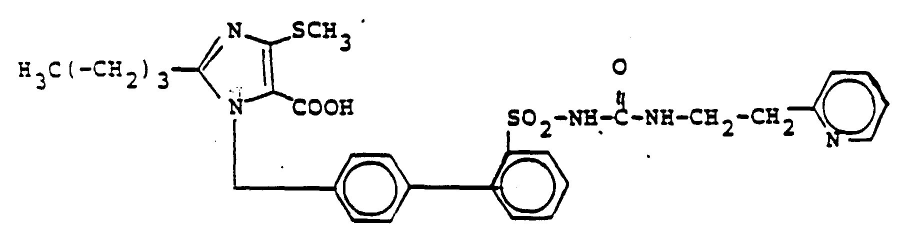
a) 1-[(2'-Ethoxycarbonylaminosulfonylbiphenyl-4-yl)methyl]-2-n-butyl-4-methylthioimidazol-5-carbonsäureethylester
   1.21 g (2.5 mmol) Verbindung 56e) und 0.78 g (5,6 mmol) Kaliumcarbonat in 20 ml trockenem DME werden zum Sieden erhitzt und 0.48 ml (5.1 mmol) Chlorameisensäureethylester zugesetzt. Nach 1 Stunde läßt man auf Raumtemperatur abkühlen und versetzt mit 50 ml 10 %iger KH₂PO₄-Lösung. Nach Extraktion mit EE wird mit Na₂SO₄ getrocknet und eingeengt. Chromatographie an SiO₂ mit EE/HEP (2/1) als Laufmittel liefert 840 mg Titelverbindung.
   R_{f} (EE/HEP 2/1) = 0.5 MS (DCI) = 559 (M⁺+H)
b) 1-[(2'-Pyridylethyl-2-aminocarbonylaminosulfonylbiphenyl-4-yl)-methyl]-2-n-butyl-4-methylthioimidazolo-5-carbonsäureethylester
   168 mg (0.3 mmol) Verbindung 57a) und 37 mg (0.3 mmol) 2-(2-Aminoethyl)-pyridin werden in 8 ml trockenem Toluol 2 Stunden zum Sieden erhitzt. Anschließend wird eingeengt und an SiO₂ chromatographiert (Laufmittel EE), wobei 34 mg der Titelverbindung anfallen.
   R_{f} (EE) = 0.15 MS (FAB) = 636 (M⁺+H)
c) Die Titelverbindung 57 wird analog zu 56f) erhalten.
   R_{f} (EE/MeOH 5/1) = 0.1 MS (FAB) = 608 (M+H)

In analoger Weise wie in Beispiel 57 beschrieben lassen sich die Verbindungen der folgenden Tabelle 4 synthetisieren.

Diese Verbindungen besitzen die Formel (C)

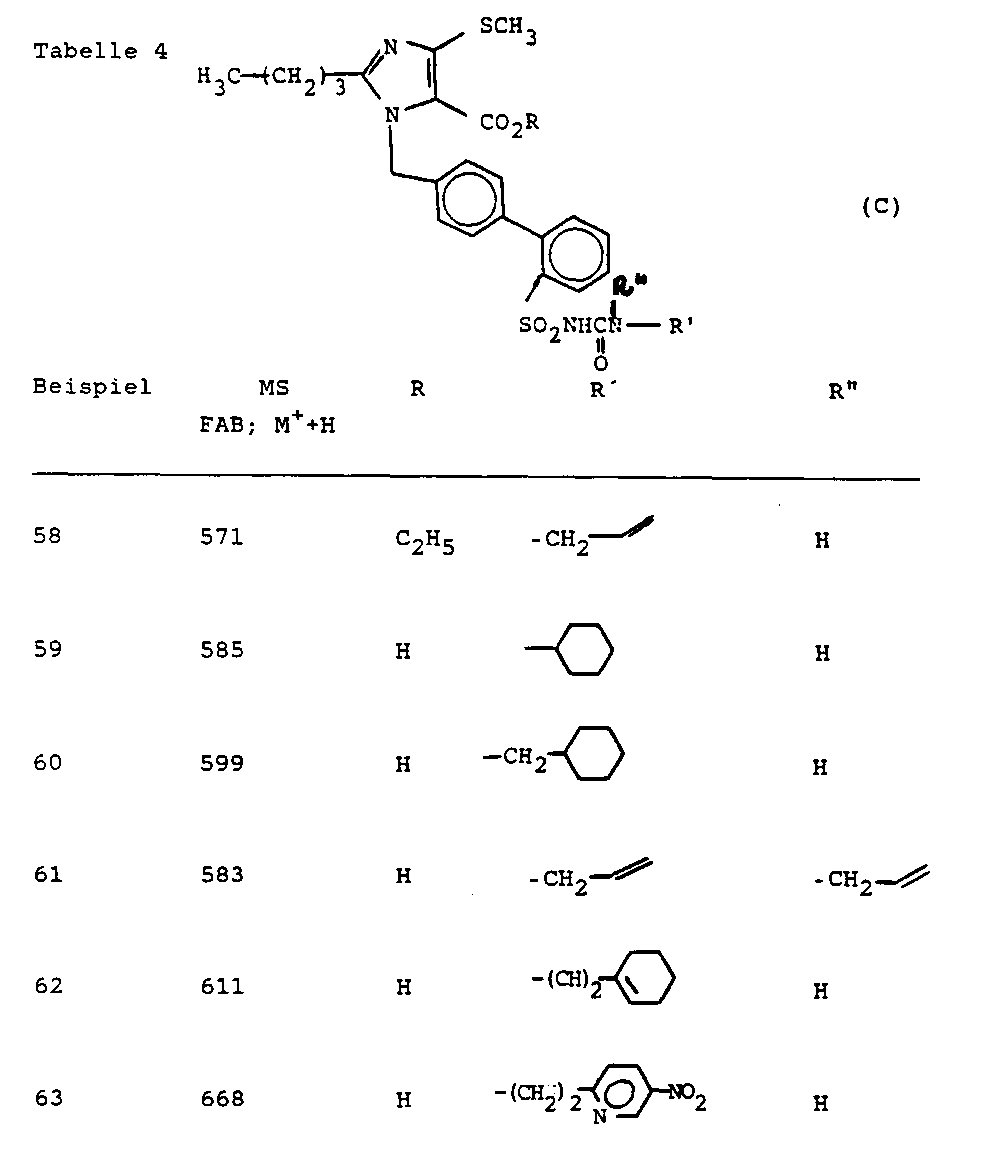

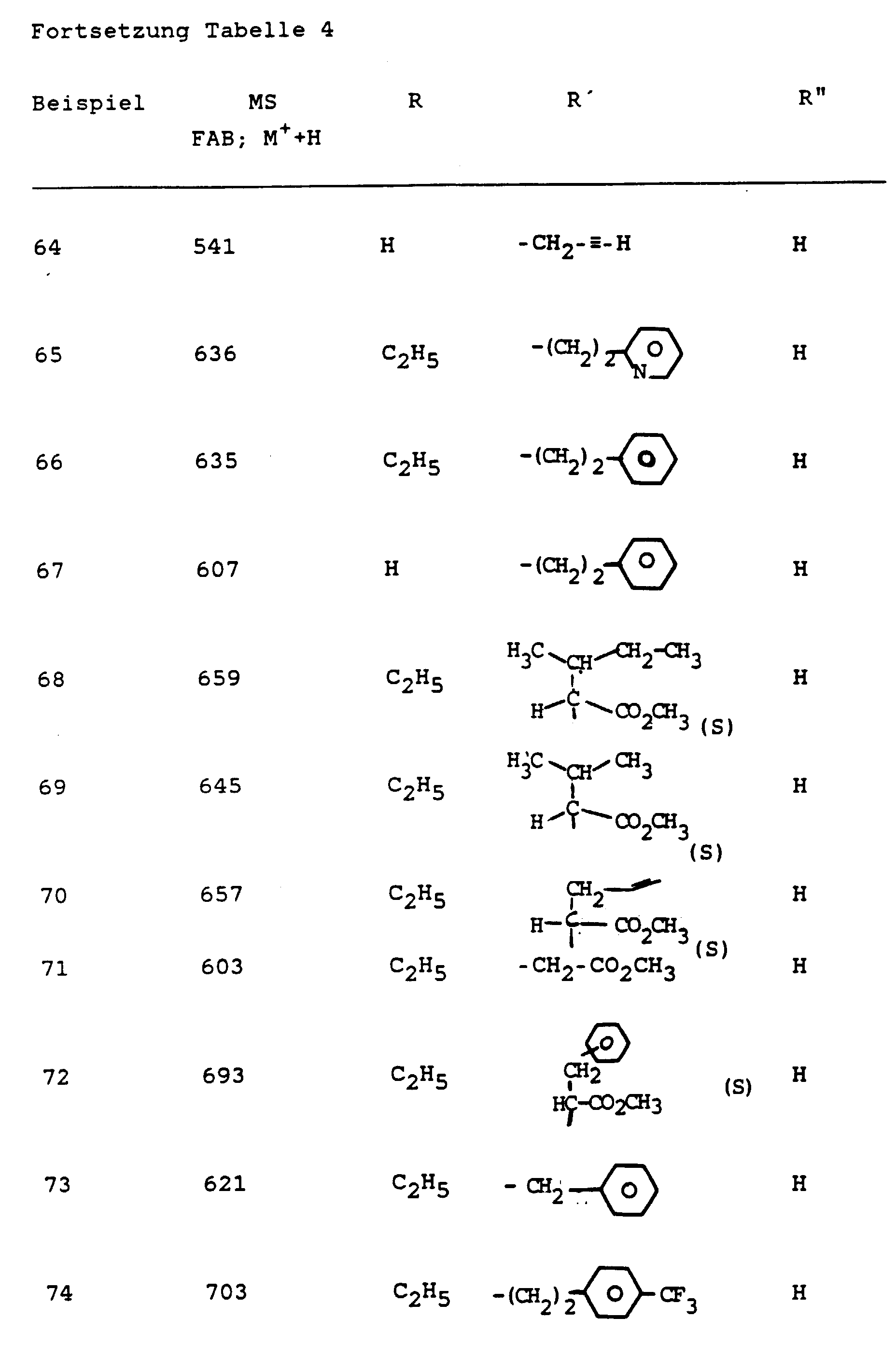

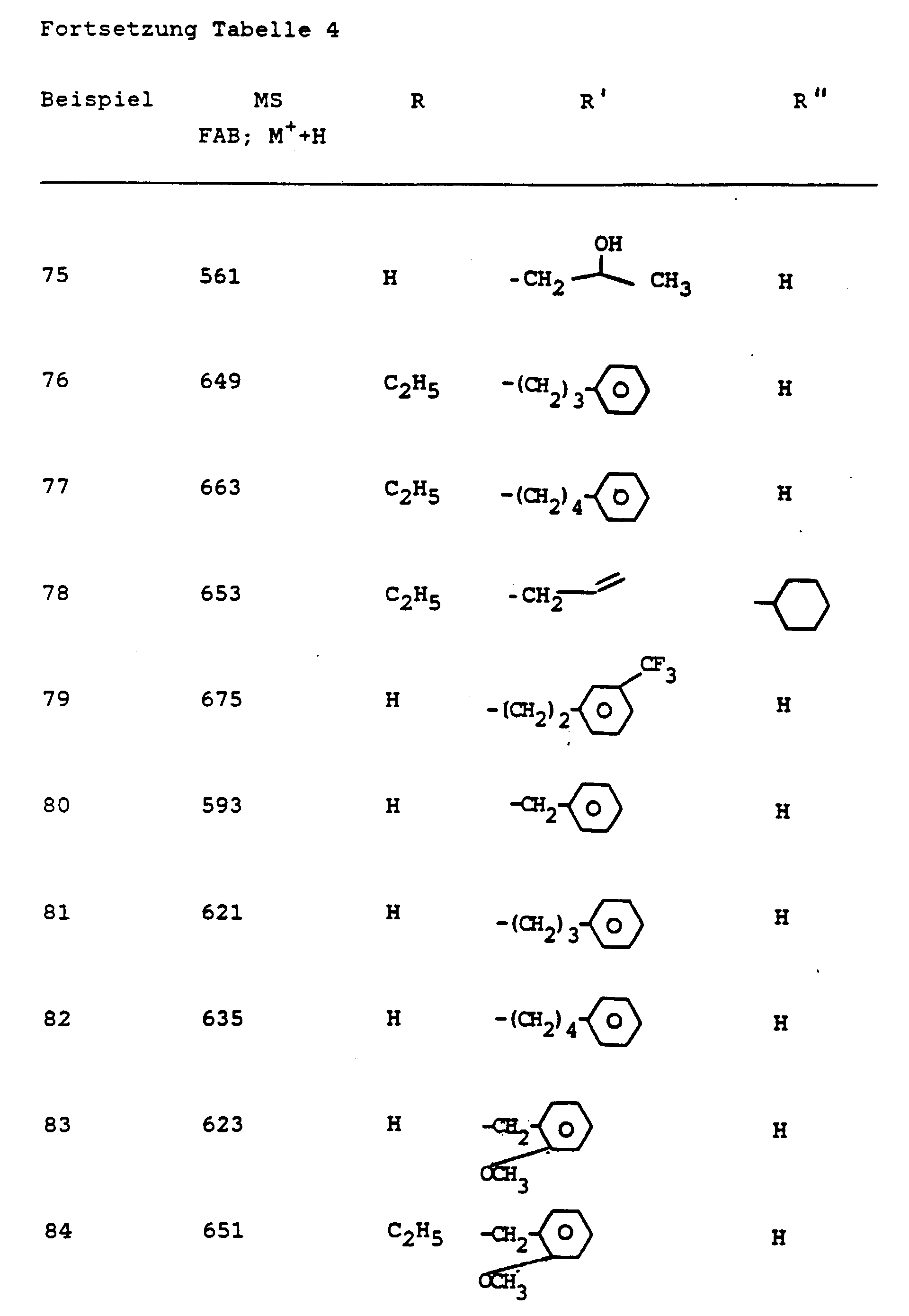

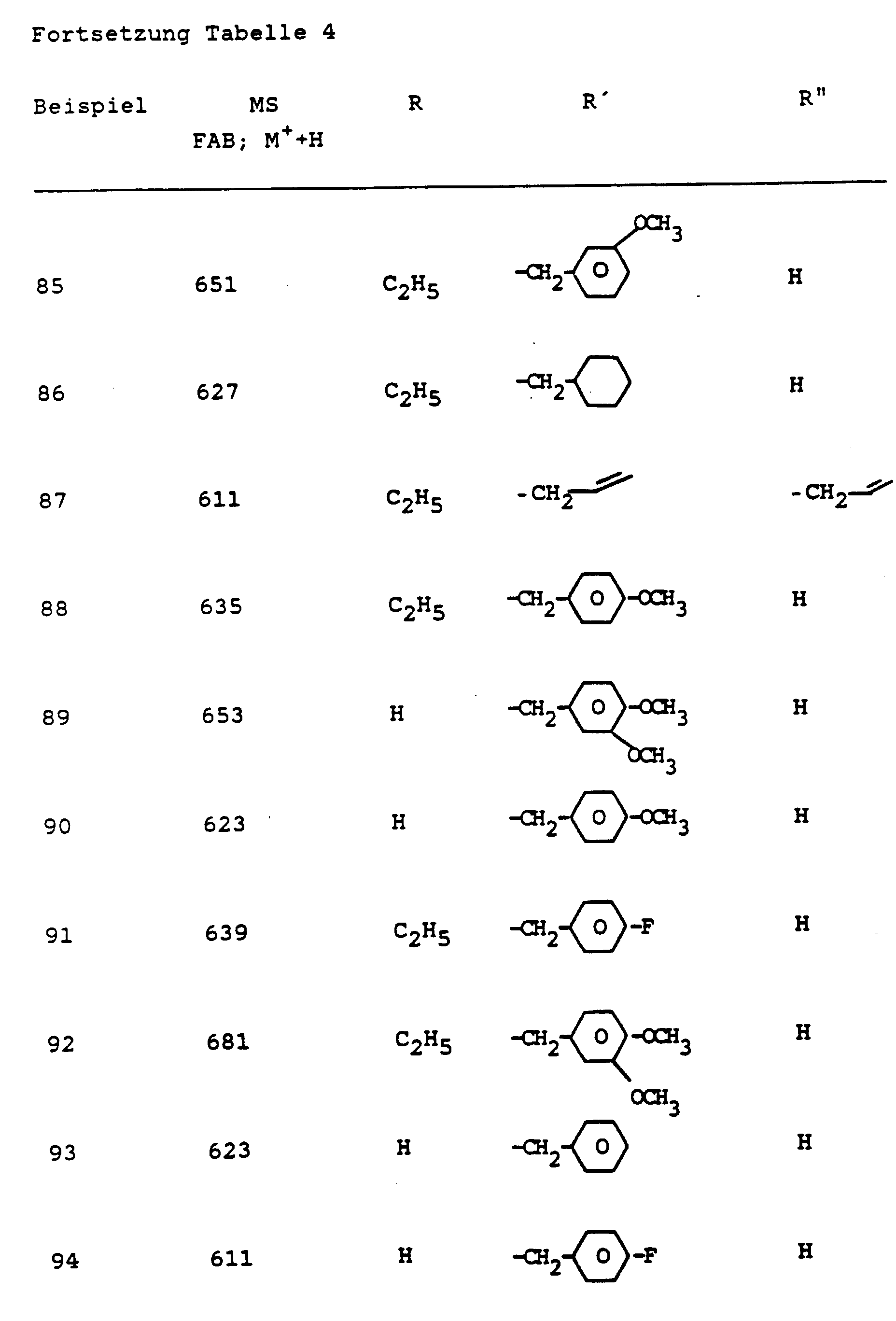

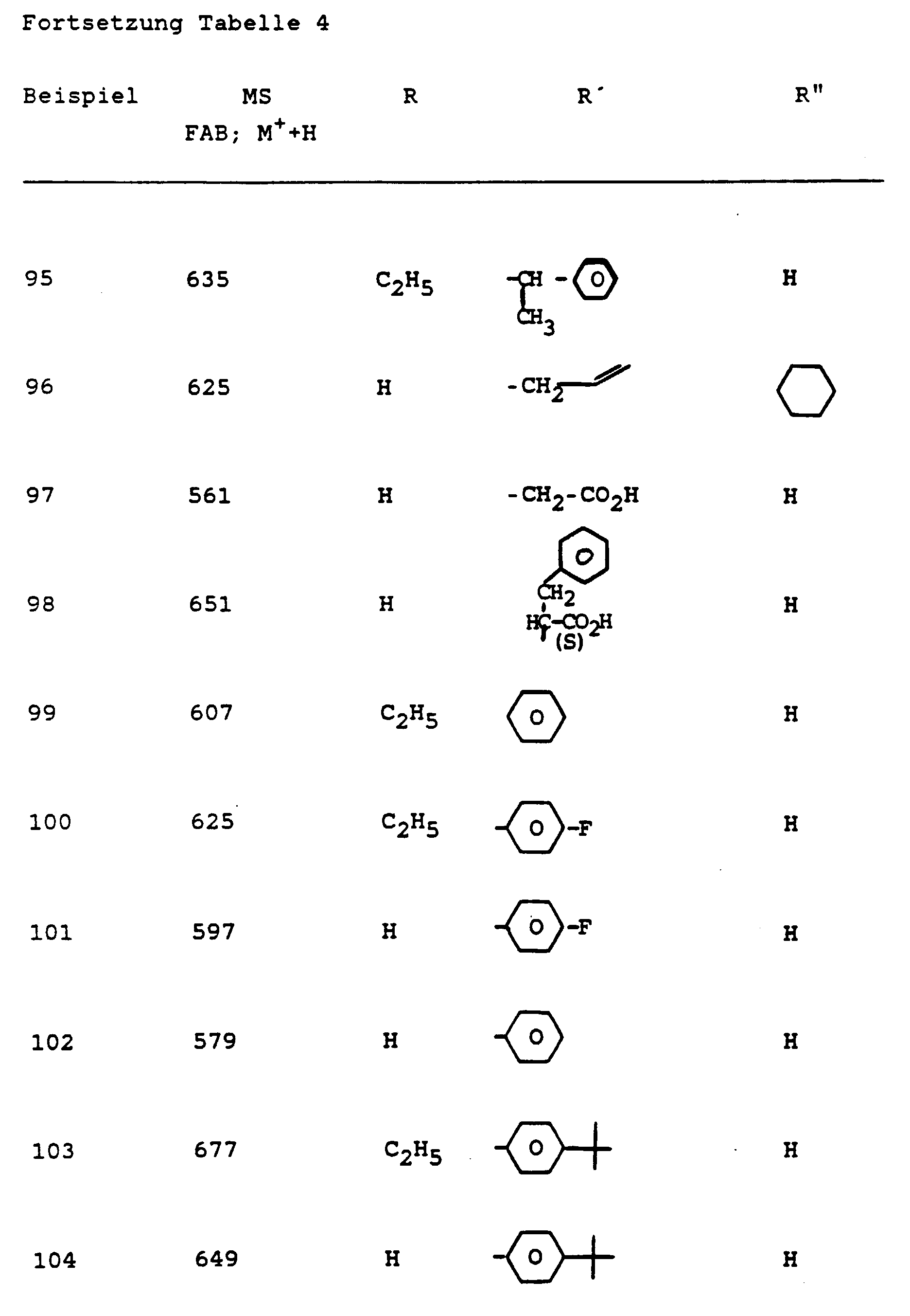

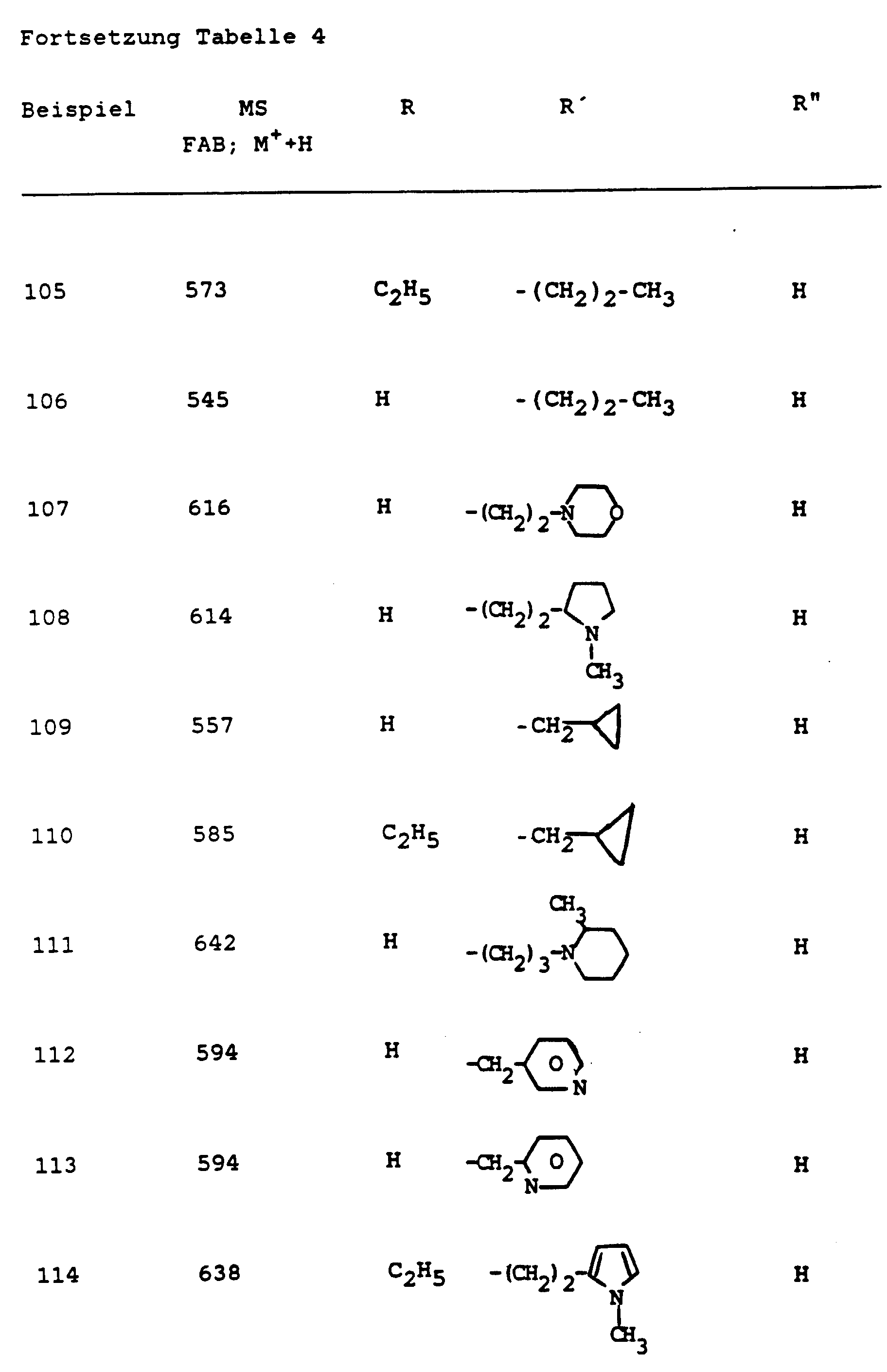

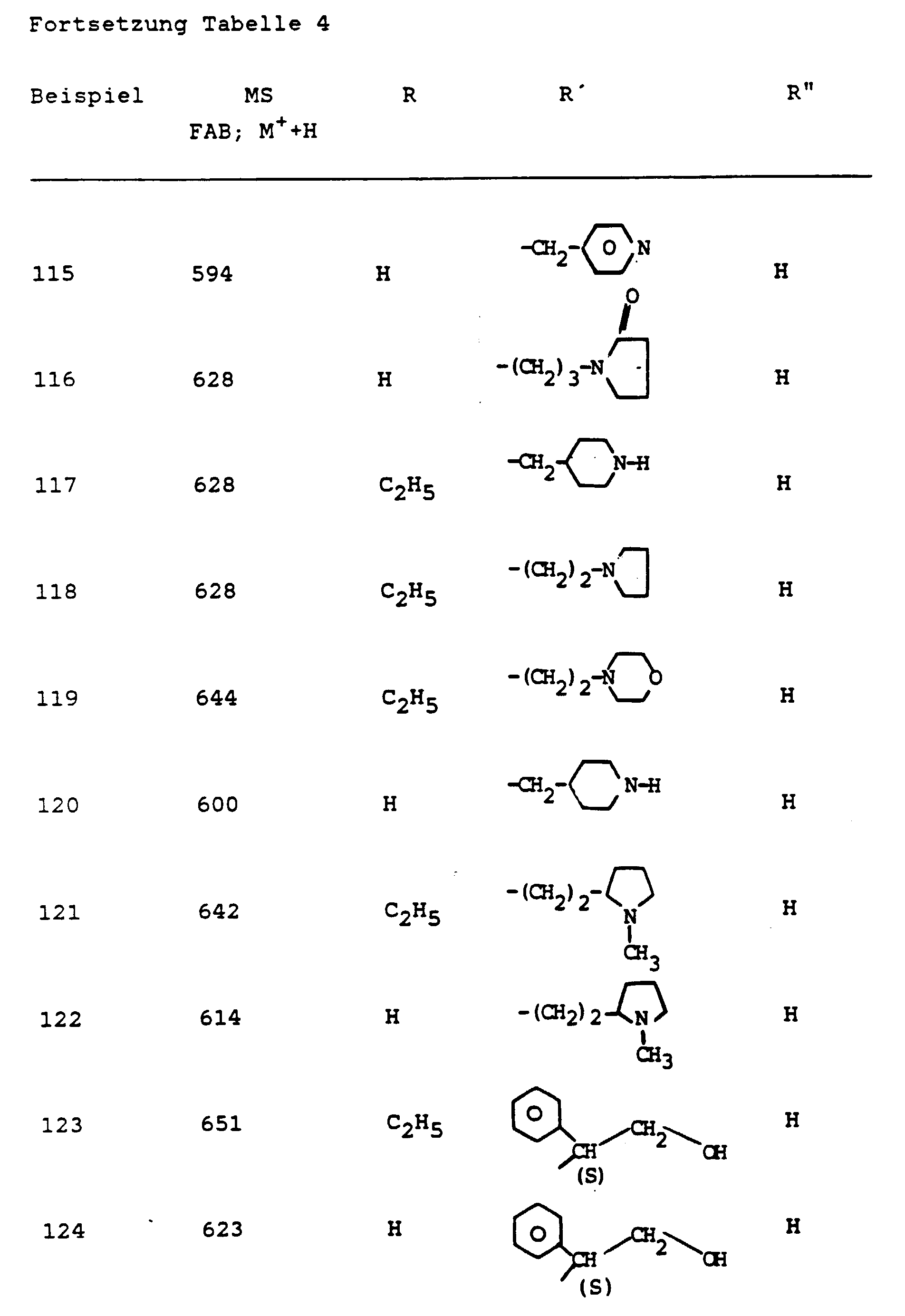

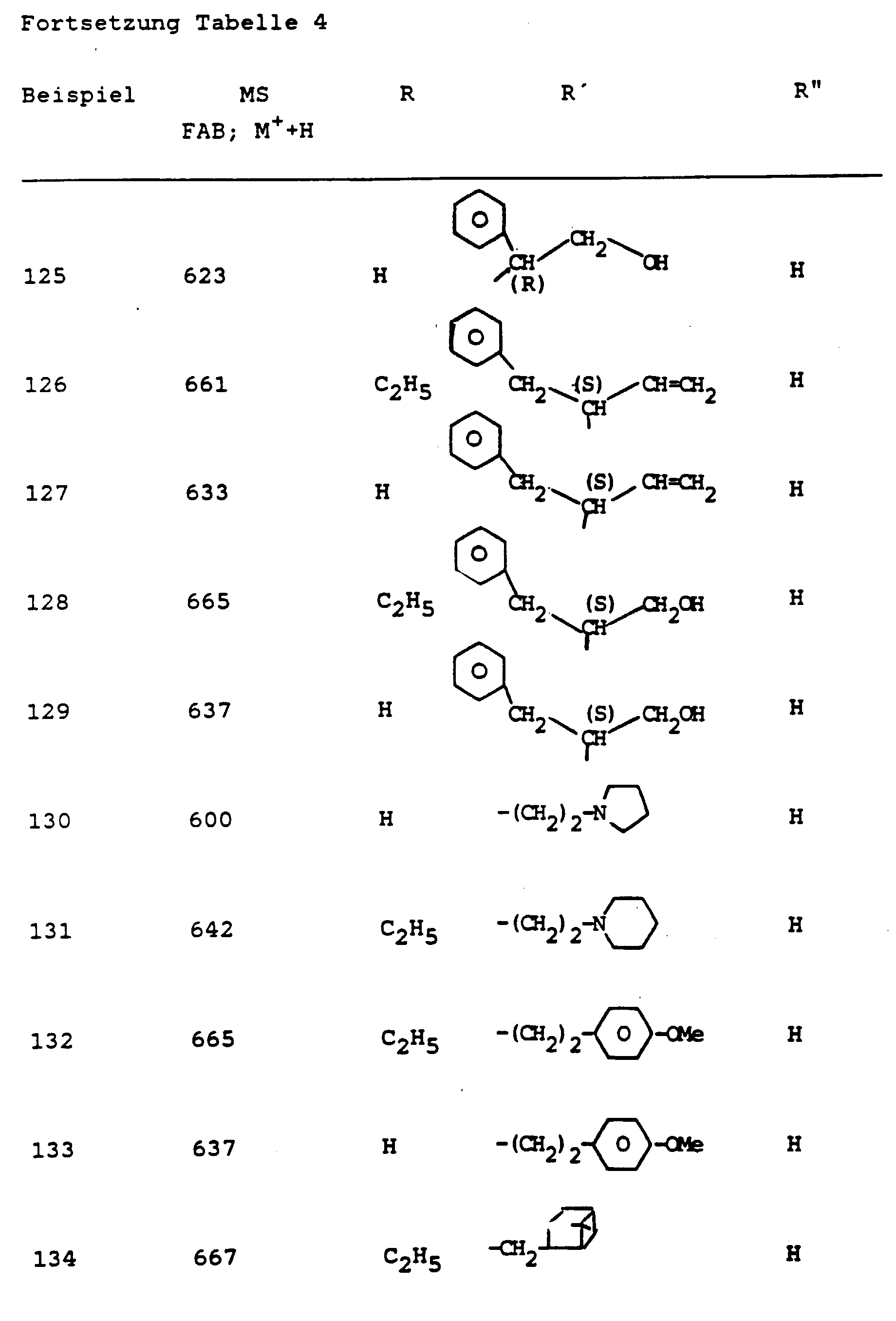

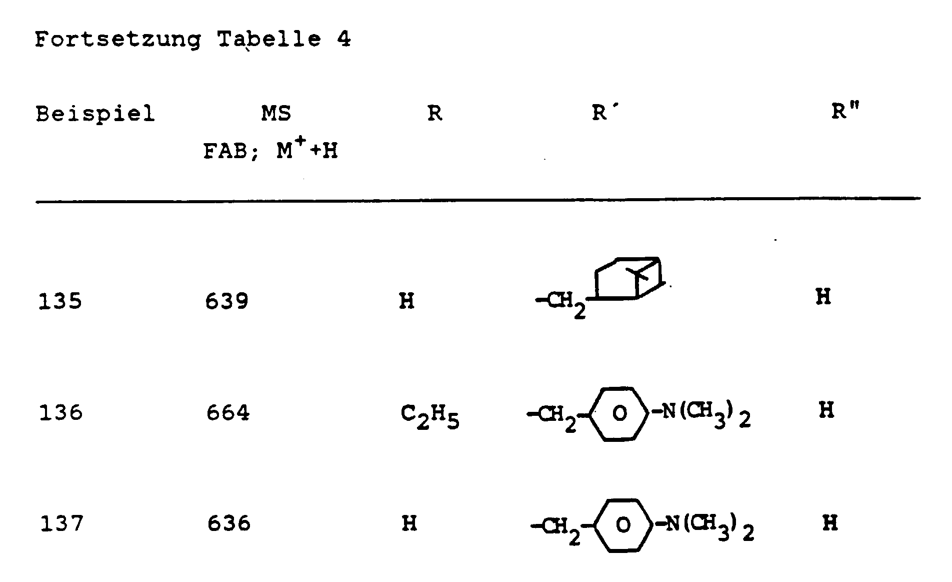

Die Verbindungen der folgenden Tabelle 5 lassen sich analog Beispiel 57 synthetisieren.

Diese Verbindungen besitzen die folgende Formel (D)

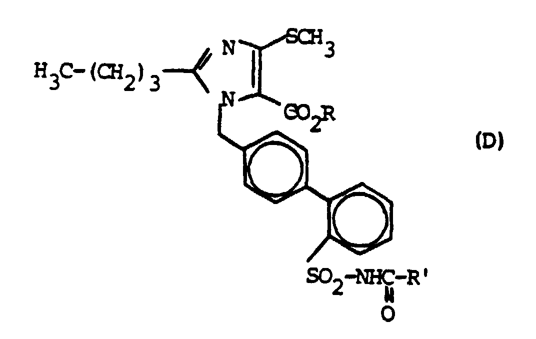

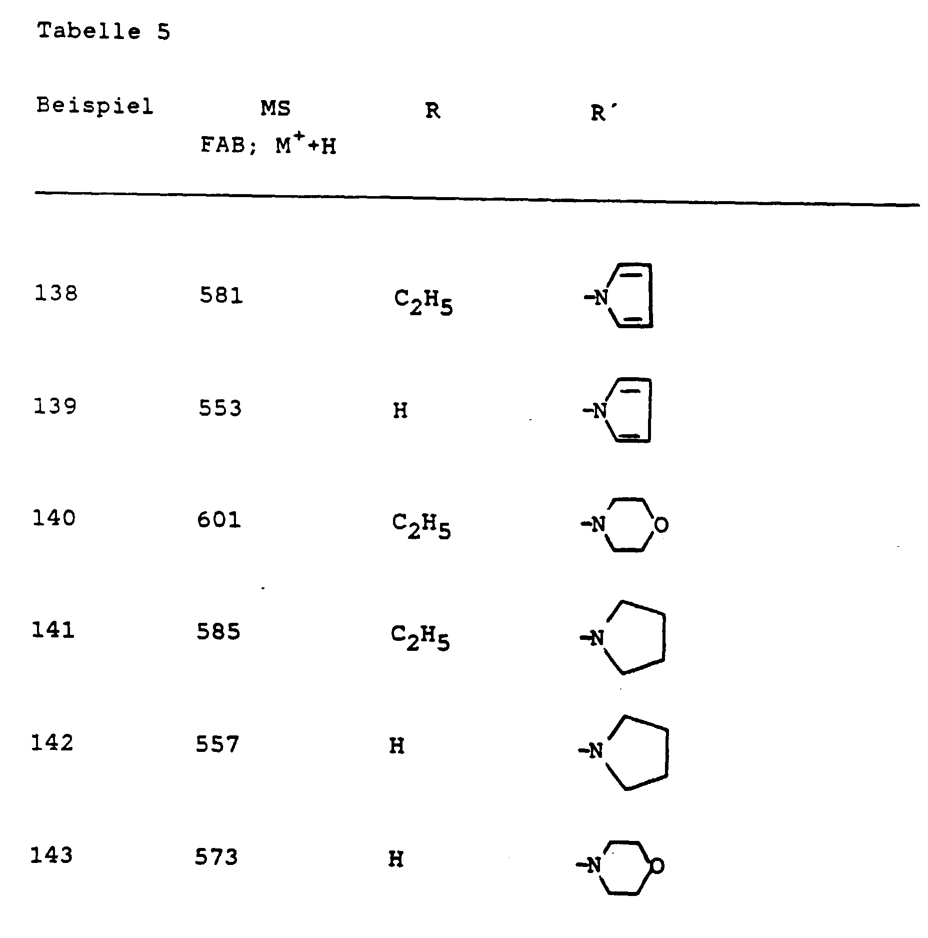

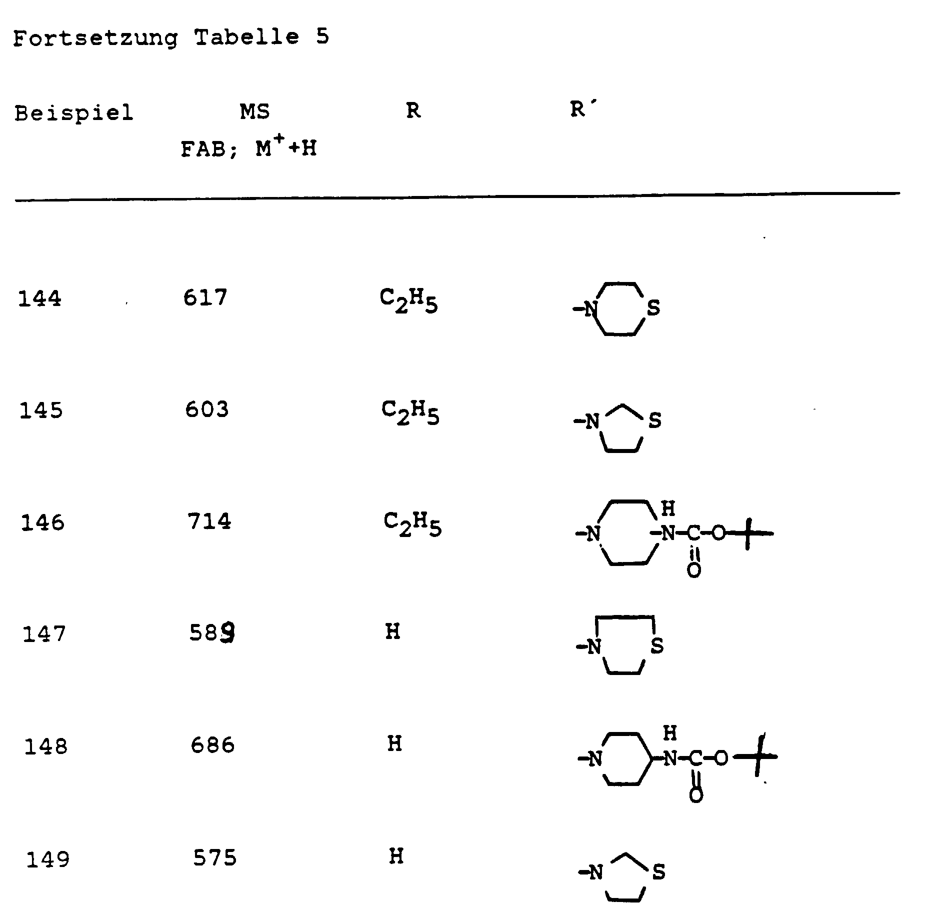

### Beispiel 150

Herstellung von 1-{[(2'-Benzoyloxycarbonylaminosulfonyl)-biphenyl-4-yl]-methyl}-2-n-butyl-4-chloro-imidazol-5-carbaldehyd

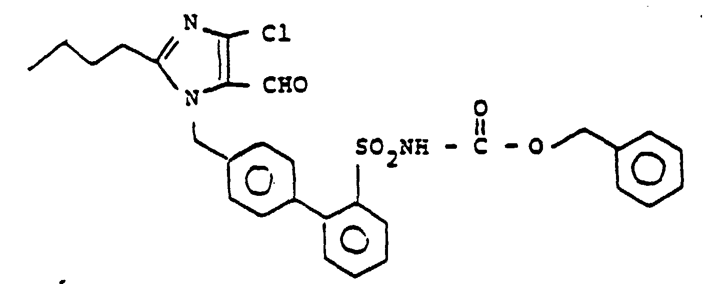

Die Titelverbindung 150 erhält man, indem 215 mg (0,5 mmol) Verbindung 1 h, 71,3 µl (0,5 mmol) Chlorameisensäure-benzylester und 70 mg (0,5 mmol) K₂CO₃ in 10 ml DMF (wasserfrei) 1,5 h unter Rückfluß erhitzt. Anschließend wird eingeengt, 100 ml EE zugegeben und je 1x mit 40 ml NaHSO₄-Lösung und NaCl-Lösung gewaschen. Die organische Phase wird getrocknet über Na₂SO₄ und einrotiert. Chromatographie mit MTB liefert 120 mg (42 %) der Titelverbindung 150 m.p. = 56 °C
R_{f} (MTC) = 0,20 MS (FAB) = 566 (M⁺+H)

Die Verbindungen der folgenden Tabelle 6 lassen sich analog Beispiel 150 bzw. 57a synthetisieren.

Diese Verbindungen besitzen die folgende Formel (E)

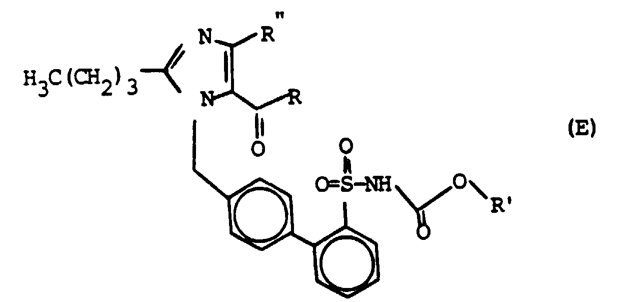

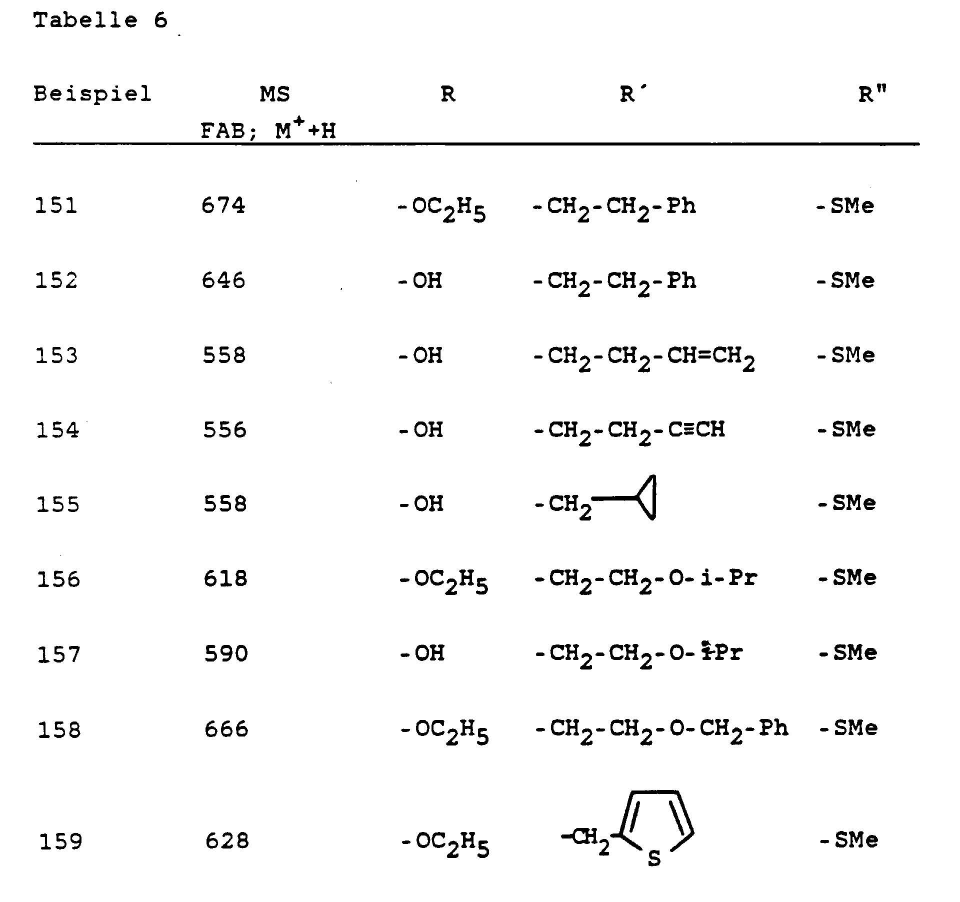

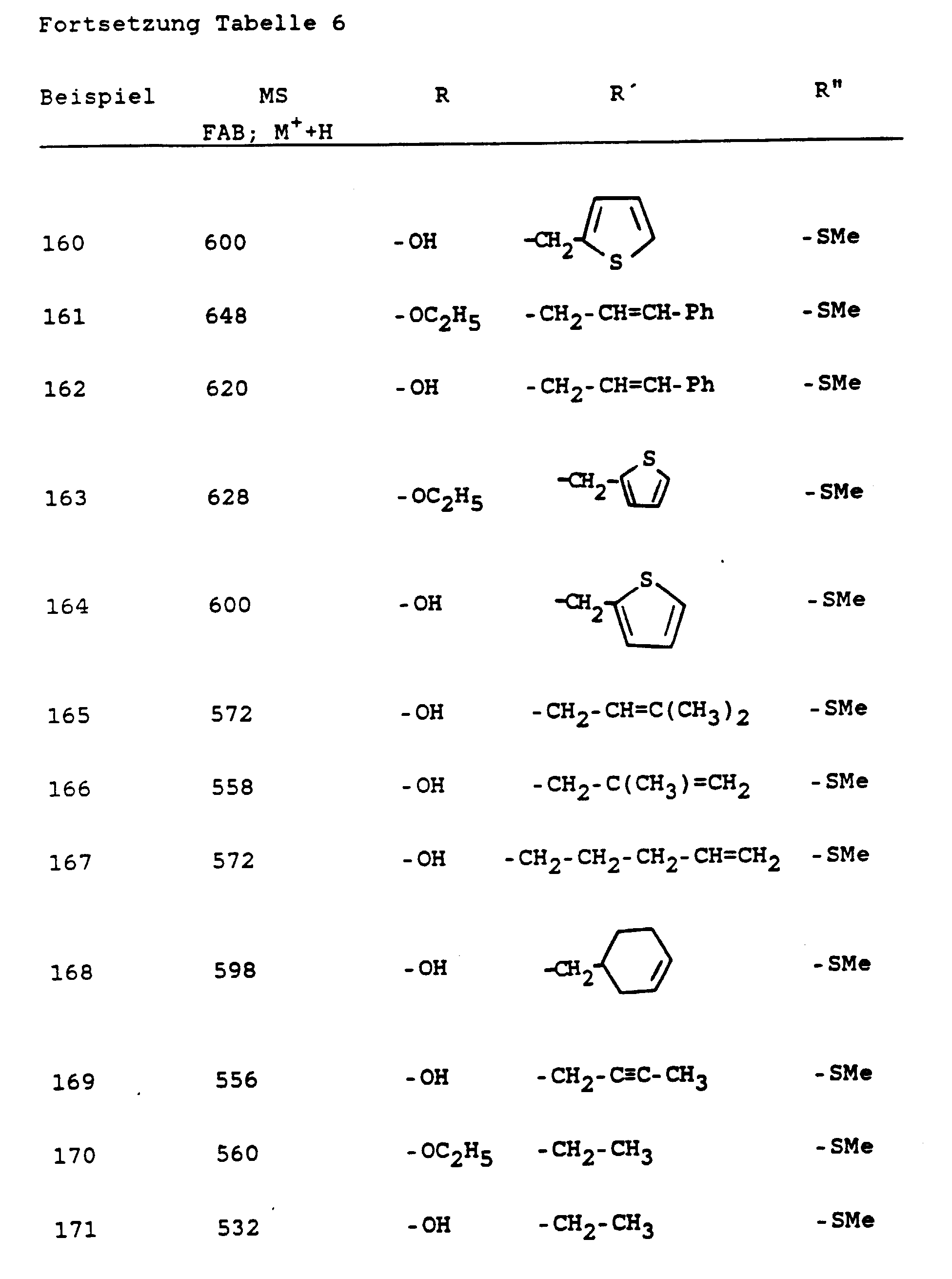

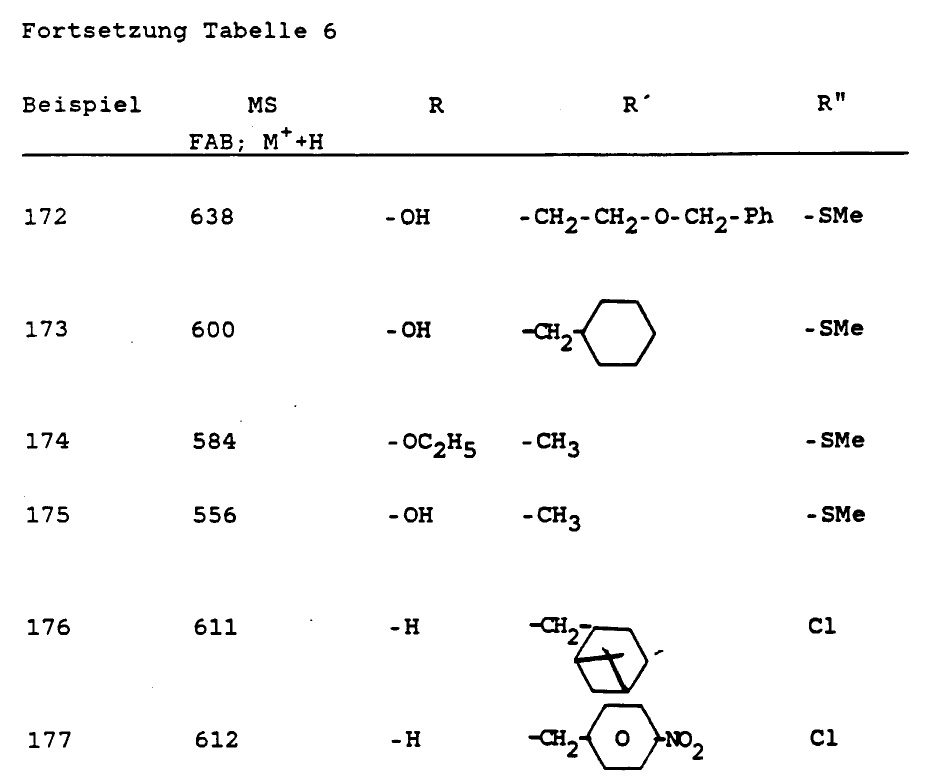

### Beispiel 178

Herstellung von 1-[(2'-Dimethylsulfamoylaminosulfonylbiphenyl-4-yl)-methyl]-2-n-butyl-4-methylthio-imidazol-5-carbonsäure-ethylester

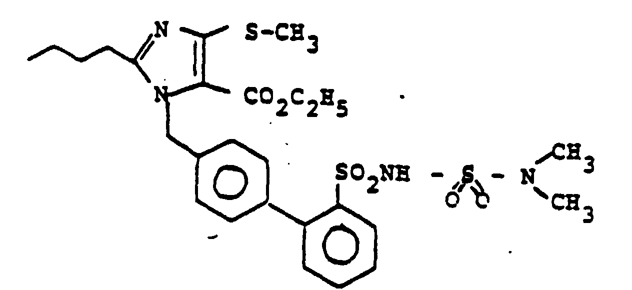

Die Titelverbindung 178 erhält man, indem man 244 mg (0,5 mmol) Verbindung 56 e), 108 µl (1,0 mmol) Sulfamoylchlorid und 140 mg (1,0 mmol) K₂CO₃ in 10 ml DME (wasserfrei) 5 Tage am Rückfluß erhitzt. Man verdünnt mit 50 ml EE und wäscht mit 50 ml KHSO₄/H₂SO₄ (pH = 1,0). Die organische Phase wird über Na₂SO₄ getrocknet und einrotiert. Chromatographie mit EE liefert 69 mg (23 %) eines farblösen Öls.
R_{f} (EE) = 0,15 MS (FAB) = 617 (M⁺+Na)

### Beispiel 179

Herstellung von 1-[1-(2'-Dimethylsulfamoylaminosulfonylbiphenyl-4-yl)-methyl]-2-n-butyl-4-methylthio-imidazol-5-carbonsäure 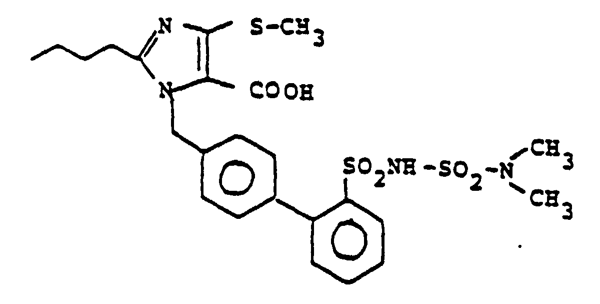 50 mg (84 µmol) der Titelverbindung des Beispiels 178 und 0,84 ml 1N NaOH werden in 3 ml Ethanol gelöst und 2 Tage bei RT gerührt. Das Ethanol wird abdestilliert, 5 ml H₂O zugegeben und mit HCl auf pH = 2 eingestellt. Der Niederschlag wird 2x mit 1 ml Wasser gewaschen und im Vakuum getrocknet. Man erhält 33 mg (70 %) eines farblosen Pulvers.
R_{f} (EE/Methanol 5:1) = 0,11 MS (FAB) = 567 (M⁺+H)

### Beispiel 180

1-[(2'-Allyloxycarbonylaminosulfonyl-biphenyl-4-yl)methyl]-2-n-butyl-4-methylthio-imidazol-5-carbonsäureethylester

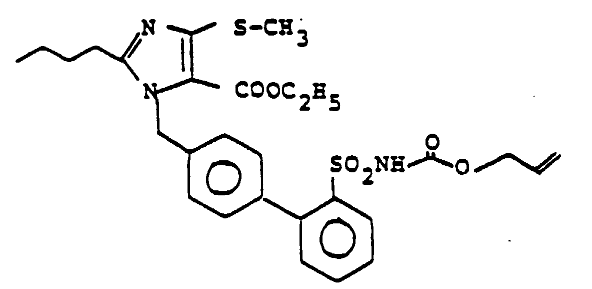

244 mg (0,5 mmol) der Verbindung 56 e), 106 µl (1,0 mmol) Chlorameisensäure-allylester und 140 mg (1,0 mmol) K₂CO₃ werden 1 h unter Rückfluß gekocht. Dann werden 50 ml 10 %ige KHSO₄-Lösung zugegeben und 3x mit je 50 ml EE extrahiert. Die organische Phase wird über Na₂SO₄ getrocknet und eingedampft. Chromatographie mit MTB/DIP 1:1 liefert 115 mg (40 %) eines farblosen Öls.
R_{f} (MTB/DIP 1:1) = 0,15 MS (FAB): 572 (M⁺+H)

### Beispiel 181

Synthese von 1-[(2'-Allyloxycarbonylaminosulfonyl-biphenyl-4-yl)methyl]-2-n-butyl-4-methylthio-imidazol-5-carbonsäure

95 mg (0,17 mmol) der Verbindung 180 werden wie unter Beispiel 179 beschrieben verseift. Man erhält 30 mg (33 %) eines farblosen Schaums.
R_{f} (EE/MeOH 10:1) = 0,1 MS (FAB) = 544

### Beispiel 182

1-[(2'-Benzyloxycarbonylaminosulfonyl-biphenyl-4-yl)methyl]-2-n-butyl-4-methylthio-imidazol-5-carbonsäure-ethylester

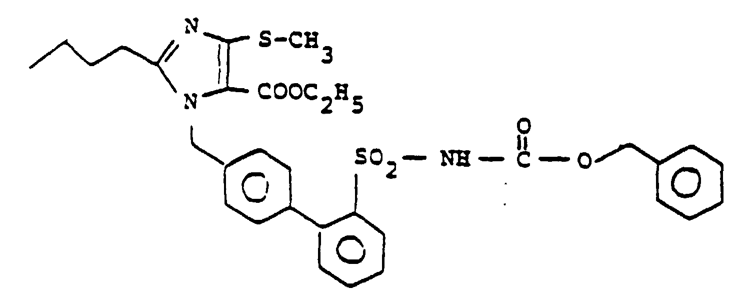

Die Titelverbindung wird analog Beispiel 180 synthetisiert.
R_{f} (MTB/DIP1:1) = 0,15 MS (FAB) = 622 (M⁺+H)

### Beispiel 183

1-[(2'-Benzyloxycarbonylaminosulfonyl-biphenyl-4-yl)methyl]-2-n-butyl-4-methylthio-imidazol-5-carbonsäure

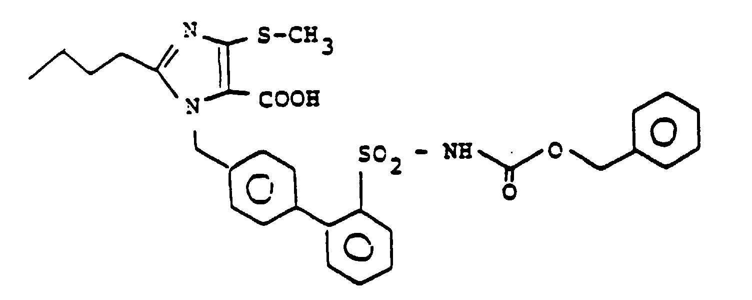

Die Titelverbindung wird analog Beispiel 181 synthetisiert.
R_{f} (EE/MeOH 10:1) = 0,1 MS (FAB) = 594 (M⁺+H)

### Beispiel 184

1-{[2'-Allylaminocarbonylaminosulfonyl)-biphenyl-4-yl-methyl}-2-n-butyl-4-methoxy-imidazol-5-carbaldehyd 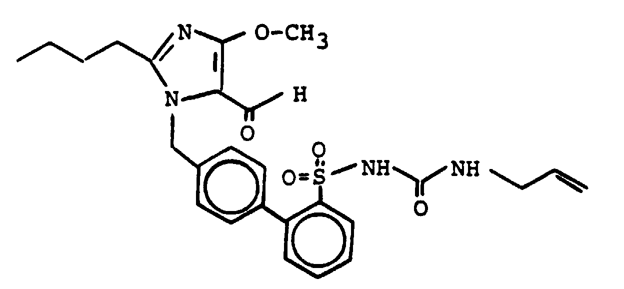
a) 1-[2'-Sulfonamidobiphenyl-4-yl)-methyl)methoxy-imidazol-5-carbaldehyd
   215 mg (0,5 mmol) Verbindung 1 h) und 1,5 mol 1N NaOH werden in 10 ml Methanol 19h unter Rückfluß gekocht. Dann wird das Methanol einrotiert, mit NaHSO₄-Lösung auf pH= 2 eingestellt und 3x mit je 50 ml EE extrahiert. Die organische Phase wird über Na₂SO₄ getrocknet und einrotiert. Chromatographie mit MTB/DIP (1:1) liefert 170 mg (80 %) der Titelverbindung,
   Schmp.: = 189°C.
   R_{f} (MTB/DIP 1:1) = 0,19 MS (DCI) = 428 (M⁺+H)
b) Die Titelverbindung 184 erhält man, indem man 150 mg (0,35 mmol) Verbindung 184a) und 3 ml Allylisocyanat 5h unter Rückfluß kocht. Anschließend wird einrotiert und mit EE chromatographiert. Man erhält 60 mg (34 %) eines farblosen Schaums.
   R_{f} (EE) = 0,34 MS (FAB) = 511 (M⁺+H)

### Beispiel 185

1-{[(2'-Ethoxycarbonylaminosulfonyl)-biphenyl-4-yl]-methyl}-2-n-butyl-4-methoxy-imidazol-5-carbaldehyd 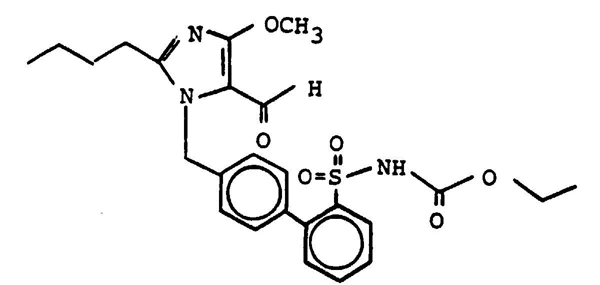 1,0 g (2,34 mmol) Verbinding 184a) werden in 50 ml Aceton (wasserfrei) gelöst und 650 mg K₂CO₃ addiert. Man erhitzt zum Rückfluß, dann wird bei dieser Temperatur 0,45 ml Chlorameisensäureethylester langsam zugespritzt. Man erhitzt weitere 4h unter Rückfluß, anschließend wird einrotiert. Mit NaHSO₄-Lösung wird auf pH= 2 angesäuert, anschließend extrahiert man 3x mit je 100 ml EE. Über Na₂SO₄ wird getrocknet, anschließend wird einrotiert und mit MTB/DIP/HOAc (15:83:2) chromatographiert. Das erhaltene Öl kann mit Diethylether kristalliert werden. Man erhält 550 mg farblose Kristalle m.p. 134°C
R_{f} (MTB) = 0,24 MS (FAB) = 500 (M⁺+H)

### Beispiel 186

1-{[(2'-Benzyloxycarbonylaminosulfonyl)-biphenyl-4-]methyl}-2-n-butyl-4-methoxy-imidazol-5-carbaldehyd

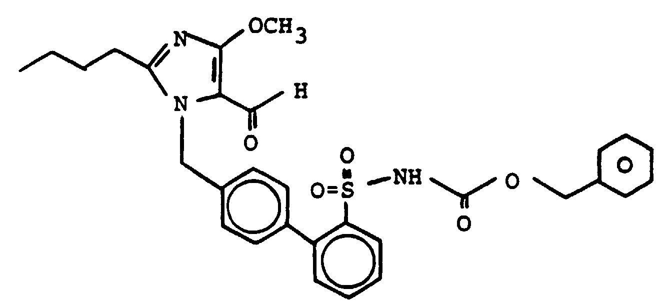

Beispiel 186 wird analog Beispiel 185 synthetisiert.
R_{f} (MTB) = 0,16 MS (FAB) = 562 (M⁺+H)

### Beispiel 187

1-{[(2'-Benzylaminocarbonylaminosulfonyl)-biphenyl-4-yl]methyl}-2-n-butyl-imidazol-5-carbonsäureethylester

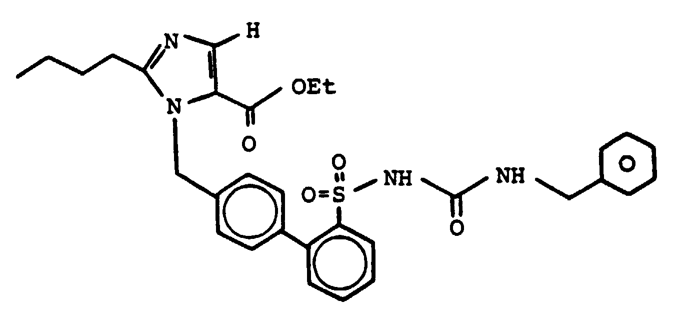

Zu 150 mg (0,24 mmol) Verbindung Beispiel 73 gelöst in 50 ml MeOH und 5 ml HOAc wird eine katalytische Menge Pd/C addiert. Das Gemisch wird in einer H₂-Atmosphäre 12h bei Raumtemperatur gerührt. Anschließend wird das Gemisch einrotiert und mit EE chromatographiert. Man erhält 30 mg (22 %) eines farblosen Schaums.
R_{f} (EE) = 0,42 MS (FAB) = 575 (M⁺+H)

### Beispiel 188

1-{[(2'-Ethoxycarbonylaminosulfonyl)-biphenyl-4-yl]-methyl}-2-n-butyl-imidazol-5-carbonsäureethylester

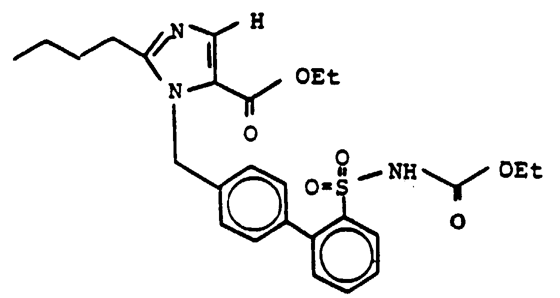

Zu 300 mg (0,5 mmol) Verbindung 57 a gelöst in 10 ml EtOH werden ca. 200 mg Raney-Nickel zugegeben. 10h wird unter Rückfluß erhitzt, weitere 200 mg Raney-Nickel zugegeben und erneut 5h unter Rückfluß erhitzt. Der Katalysator wird abfiltriert und das Solvens einrotiert. Der Rücktand wird mit MTB chromatographiert und man erhält 50 mg (18 %) eines farblosen Schaums.
R_{f} (EE) = 0,27 MS (FAB) = 514 (M⁺+H)

### Beispiel 189

1-[(2'-{2-Thienylsulfonylaminosulfonyl}-biphenyl-4-yl)-methyl]-2-n-butyl-4-methylthio-imidazol-5-carbonsäureethylester 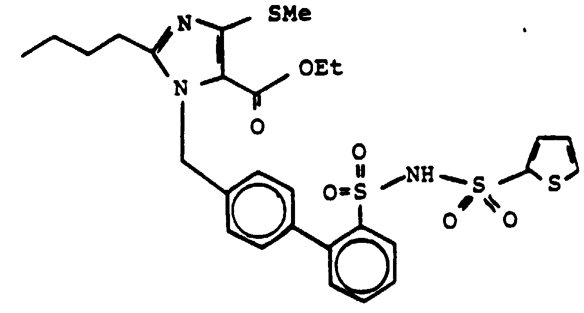 244 mg (0,5 mmol) Sulfonamid des Beispiels 56e werden in 10 ml Diethylenglykoldimethylether (wasserfrei) gelöst. Anschließend werden 346 mg (2,5 mmol) K₂CO₃ sowie 81 mg (0,5 mmol) 2-Thienylsulfonylchlorid zugegeben. 2h wird unter Rückfluß erhitzt, dann gießt man das abgekühlte Reaktionsgemisch in 50 ml 5 % NaHSO₄-Lösung und extrahiert 3x mit je 50 ml EE. Über Na₂SO₄ wird getrocknet, einrotiert und mit EE chromatographiert. Man erhält 310 mg blaßgelber Kristalle, m.p. = 120 - 122°C.
R_{f} (EE) = 0,24 MS (FAB) = 634 (M⁺+H)

### Beispiel 190

1-[(2'-{2-Thienylsulfonylaminosulfonyl}-biphenyl-4-yl) methyl]-2-n-butyl-4-methylthio-imidazol-5-carbonsäure

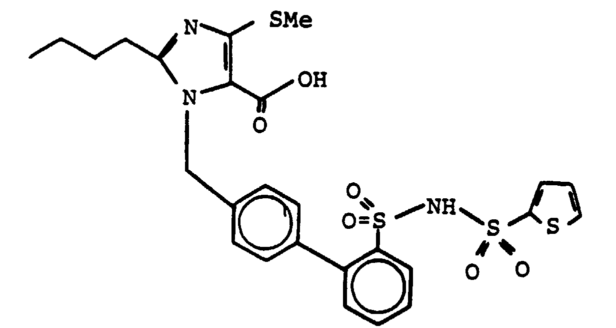

Die Verseifung des Ethylesters aus Beispiel 189 erfolgt analog Beispiel 56f
R_{f} (EE/MeOH 5:1) = 0,13 MS (FAB) = 606 (M⁺+H)

Die Verbindungen der folgenden Tabelle 7 lassen sich analog Beispiel 189 bzw. analog Beispiel 190 (bzw. 56f) synthetisieren.

Diese Verbindungen besitzen die folgende Formel F)

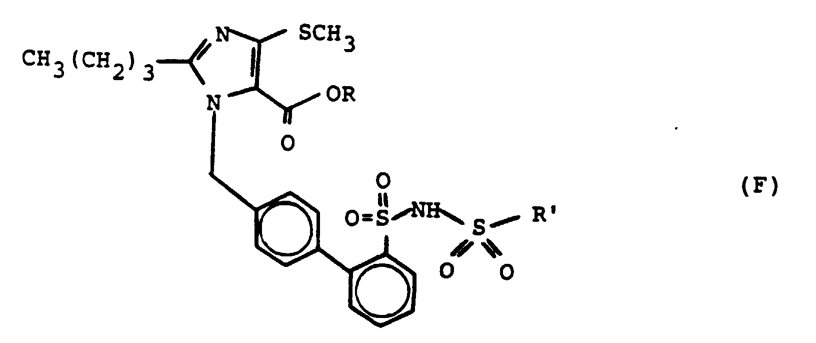

**Tabelle 7**

| Beispiel | MS(FAB) M⁺+H | R | R' |
|---|---|---|---|
| 191 | 673 | C₂H₅ | 4-Nitrophenyl |
| 192 | 645 | H | " |
| 193 | 579 | C₂H₅ | C₂H₅ |
| 194 | 634 | C₂H₅ | 2-Thienyl |

### Beispiel 195

### 1-[2'-Methylaminocarbonylaminosulfonylbiphenyl-4-yl)-methyl]-2-n-butyl-4-methylthio-imidazol-5-carbonsäureethylester

In einem Autoklaven werden zu 1 g Sulfonylcarbamat aus Beispiel 57 a in 50 ml Toluol bei 80°C 5 min lang Methylamin eingeleitet. Nun erhitzt man 8h auf 80°C. Danach wird im Vakuum eingeengt und der Rückstand an Kieselgel chromatographiert (EE/n-HEP 2/1), wobei die Titelverbindung als amorphes Pulver anfällt.
R_{f} (EE/n-HEP 2/1) = 0,1 MS (FAB) = 545 (M⁺+H)

In analoger Weise wie in Beispiel 195 bzw. Beispiel 56f können die Verbindungen der folgenden Tabelle 8 synthetisiert werden (die Verbindung aus Beispiel 195 ist ebenfalls aufgeführt).

Diese Verbindungen besitzen die Formel C

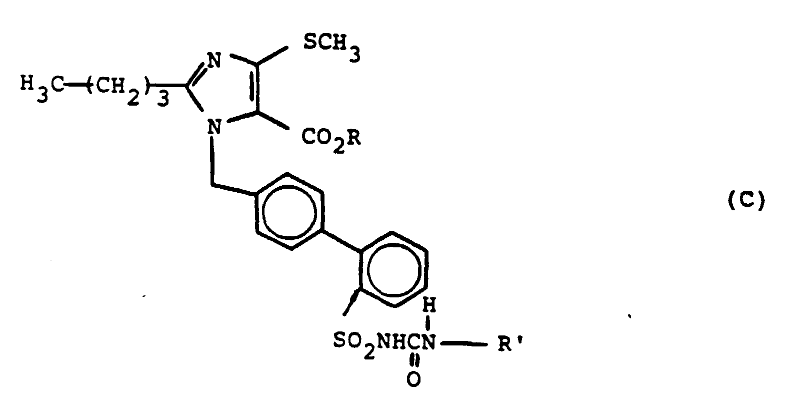

**Tabelle 8**

| Beispiel | MS (FAB); M⁺+H | R | R' |
|---|---|---|---|
| 195 | 545 | -C₂H₅ | -CH₃ |
| 196 | 517 | H | -CH₃ |
| 197 | 559 | -C₂H₅ | -C₂H₅ |
| 198 | 531 | H | -C₂H₅ |
| 199* | 619 | -C₂H₅ | -CH₂-CH₂-O-CH₂-CH₂-OH |
| 200* | 591 | H | -CH₂-CH₂-O-CH₂-CH₂-OH |

| | | | |
|---|---|---|---|
| *Die Verbindungen werden analog Beispiel 57 synthetisiert. | | | |

## Patentansprüche (Patentansprüche für folgende(n) Vertragsstaat(en): AT, BE, CH, DE, DK, FR, GB, IT, LI, LU, NL, PT, SE)

1. Verbindung der Formel (I), 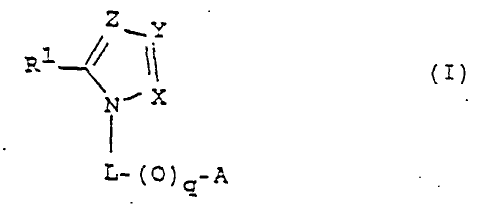 in welcher die Symbole folgende Bedeutungen tragen:
Z Stickstoff
X,Y unabhängig voneinander CR²
R¹ (C₂-C₇)-Alkyl, (C₃-C₇)-Alkenyl oder (C₃-C₇)-Alkinyl,
R² Wasserstoff, Halogen, Nitro, (C₁-C₃)-Perfluoralkyl, Cyano, (C₁-C₁₀)-Alkyl, (C₃-C₁₀)-Alkenyl, -CH₂OR⁵, -S(O)ᵣ-R¹⁹, -CO-R⁸ oder -O-R⁶,
R³
1. Wasserstoff,
2. (C₁-C₈)-Alkyl,
3. (C₃-C₈)-Cycloalkyl,
4. Phenyl,
5. Benzyl oder
6. den unter 2. definierten Rest, worin 1 bis alle H-Atome durch Fluor substituiert sind;
R⁵ Wasserstoff oder (C₁-C₆)-Alkyl
R⁶, R⁹
1. Wasserstoff,
2. (C₁-C₆)-Alkyl, das mit 1-3 Resten aus der Reihe (C₁-C₆)-Alkoxy, das seinerseits mit 1-3 Resten aus der Reihe Hydroxy, (C₁-C₆)-Alkoxy, Amino, Mono-(C₁-C₆)-Alkylamino, Di-(C₁-C₆)-Alkylamino substituiert sein kann, (C₂-C₁₀)-Alkenyl, Hydroxy, Amino, Mono-(C₁-C₆)-Alkylamino, Di-(C₁-C₆)-Alkylamino, (C₁-C₆)-Alkoxycarbonylamino, (C₆-C₁₂)-Aryl-(C₁-C₄)-Alkoxycarbonylamino; (C₆-C₁₀)-Aryl, (C₆-C₁₀)Aryl- (C₁-C₃)-Alkyl, (C₁-C₉)-Heteroaryl, Carboxy und (C₁-C₄)-Alkoxycarbonyl substituiert sein kann;
3. (C₃-C₈)-Cycloalkyl,
4. (C₃-C₆)-Cycloalkyl-(C₁-₃)alkyl,
5. (C₆-C₁₂)-Aryl, vorzugsweise Phenyl,
6. (C₆-C₁₀)-Aryl-(C₁-C₄)-Alkyl,
7. (C₁-C₉)-Heteroaryl, welches teilweise oder vollständig hydriert sein kann,
8. (C₁-C₉)-Heteroaryl-(C₁-C₃)-alkyl, wobei der Heteroarylteil teilweise oder vollständig hydriert sein kann,
9. einen wie vorstehend unter 5., 6., 7. und 8. definierten Rest, substituiert mit 1 oder 2 gleichen oder verschiedenen Resten aus der Reihe Halogen, (C₁-C₄)-Alkyl, Hydroxy, Methoxy, Nitro, Cyano, CO₂R³, Trifluormethyl, -NR¹¹R¹² und 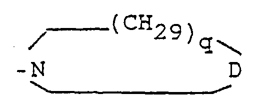
10. (C₁-C₆)-Alkyl, worin 1 bis alle H-Atome durch Fluor substituiert sind,
11. (C₂-C₆)-Alkenyl oder (C₃-C₆)-Alkenoyl,
12. (C₃-C₈)-Cycloalkenyl,
13. (C₃-C₈)-Cycloalkenyl-(C₁-C₃)-Alkyl,
14. (C₆-C₁₀)-Aryl-(C₁-C₄)-Alkyl,
15. (C₆-C₁₀)-Aryl-(C₃-C₆)-Alkenyl,
16. (C₁-C₉)-Hetaryl-(C₃-C₆)-Alkenyl,
17. (C₃-C₆)-Alkinyl,
18. (C₆-C₁₀)-Aryl-(C₃-C₆)-Alkinyl,
19. (C₁-C₉)-Hetaryl-(C₃-C₆)-Alkinyl,
20. R⁶, R⁹ gemeinsam mit dem sie tragenden N-Atom ein Hetaryl, das auch teilweise oder vollständig hydriert sein kann,
R⁷ Wasserstoff
R⁸ Wasserstoff oder -OR⁶
R¹¹,R¹² unabhängig voneinander Wasserstoff oder (C₁-C₄)-Alkyl
D -NR¹³, -O oder -CH₂
R¹³ Wasserstoff oder (C₁-C₄)-Alkyl
A Biphenylrest der mit einem Rest R¹⁵ oder mit R¹⁴ und R¹⁵ zusammen substituiert ist
R¹⁵ -SO₂-NR⁷-CO-NR⁶R⁹, -SO₂-NH-COO-R⁶, -SO₂-NH-SO₂-NR⁶R⁹, -SO₂-NH-CO-R⁶ oder -SO₂-NH-SO₂-R⁶; oder
R¹⁴ und R¹⁵ zusammen -CO-NH-SO₂- sein kann
R¹⁹
1. (C₁-C₆)-Alkyl
2. (C₃-C₈)-Cycloalkyl
3. Phenyl
4. Benzyl oder
5. den unter 1. definierten Rest, worin ein bis alle H-Atome durch Fluor substituiert sind
L -CH₂-
q Null und
r Null, 1 oder 2,
sowie deren physiologisch verträgliche Salze.

2. Verbindung der Formel I gemäß Anspruch 1, dadurch gekennzeichnet, daß die Verbindung Ethyl 1-[2'-n-Propylaminocarbonylaminosulfonylbiphenyl-4-yl) Methyl]-2-n-Butyl-4-Methylthioimidazol-5-Carboxylat ist oder dessen physiologisch verträglichen Salze.

3. Verbindung der Formel I gemäß Anspruch 1, dadurch gekennzeichnet, daß die Verbindung 1-[2'-n-Propylaminocarbonylaminosulfonylbiphenyl-4yl)Methyl]-2 -n-Butyl-4-Methylthioimidazol-5-Carbonsäure ist oder dessen physiologisch verträglichen Salze.

4. Verfahren zur Herstellung einer Verbindung der Formel (I) gemäß einem der Ansprüche 1 bis 3, sowie von deren physiologisch verträglichen Salzen, dadurch gekennzeichnet, daß man eine Verbindung der Formel (II) 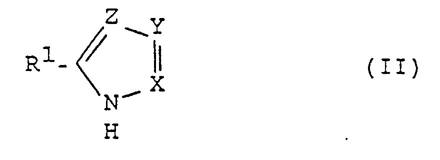 worin R¹, X, Y und Z wie in Anspruch 1 definiert sind, alkyliert mit Verbindungen der Formel (III),
U-L-(O)_{q}-A (III)
worin L, A und q wie in Anspruch 1 definiert sind, und U für eine Fluchtgruppe steht, gegebenenfalls temporär eingeführte Schutzgruppen wieder abspaltet, erhaltene Sulfonamide der Formel I gegebenenfalls in Urethane der Formel I überführt, erhaltene Sulfonamide der Formel I oder erhaltene Urethane der Formel I gegebenenfalls in Sulfonylharnstoffe der Formel I überführt, und die erhaltenen Verbindungen der Formel (I) gegebenenfalls in ihre physiologisch verträglichen Salze überführt.

5. Verbindung gemäß einem der Ansprüche 1 bis 3 zur Anwendung als Heilmittel.

6. Verbindung gemäß einem der Ansprüche 1 bis 3 zur Anwendung als Heilmittel zur Behandlung des Bluthochdrucks.

7. Pharmazeutische Zubereitung enthaltend eine Verbindung gemäß einem der Ansprüche 1 bis 3.

8. Verfahren zur Herstellung einer Zubereitung gemäß Anspruch 7, dadurch gekennzeichnet, daß man eine Verbindung der Formel (I) gemäß Anspruch 1 zusammen mit einem physiologisch unbedenklichen Träger und gegebenenfalls weiteren Zusatz- oder Hilfsstoffen in eine geeignete Darreichungsform bringt.

## Patentansprüche (Patentansprüche für folgende(n) Vertragsstaat(en): ES, GR)

1. Verfahren zur Herstellung von Verbindungen der Formel I 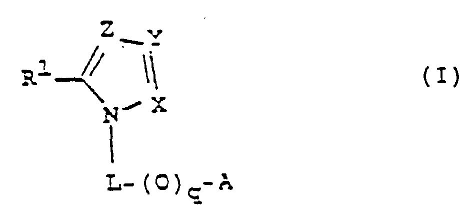 in welcher die Symbole folgende Bedeutungen tragen:
Z Stickstoff
X,Y unabhängig voneinander CR²
R¹ (C₂-C₇)-Alkyl, (C₃-C₇)-Alkenyl oder (C₃-C₇)-Alkinyl,
R² Wasserstoff, Halogen, Nitro, (C₁-C₃)-Perfluoralkyl, Cyano, (C₁-C₁₀)-Alkyl, (C₃-C₁₀)-Alkenyl, -CH₂OR⁵, -S(O)ᵣ-R¹⁹, -CO-R⁸ oder O-R⁶,
R³
1. Wasserstoff,
2. (C₁-C₈)-Alkyl,
3. (C₃-C₈)-Cycloalkyl,
4. Phenyl,
5. Benzyl oder
6. den unter 2. definierten Rest, worin 1 bis alle H-Atome durch Fluor substituiert sind;
R⁵ Wasserstoff oder (C₁-C₆)-Alkyl
R⁶, R⁹
1. Wasserstoff,
2. (C₁-C₆)-Alkyl, das mit 1-3 Resten aus der Reihe (C₁-C₆)-Alkoxy, das seinerseits mit 1-3 Resten aus der Reihe Hydroxy, (C₁-C₆)-Alkoxy, Amino, Mono-(C₁-C₆)-Alkylamino, Di-(C₁-C₆)-Alkylamino substituiert sein kann, (C₂-C₁₀)-Alkenyl, Hydroxy Amino, Mono-(C₁-C₆)-Alkylamino, Di-(C₁-C₆)-Alkylamino, (C₁-C₆)-Alkoxycarbonylamino, (C₆-C₁₂)-Aryl-(C₁-C₄)-Alkoxycarbonylamino, (C₆-C₁₀)-Aryl, (C₆-C₁₀)Aryl-(C₁-C₃)-Alkyl, (C₁-C₉)-Heteroaryl, Carboxy und (C₁-C₄)-Alkoxycarbonyl substituiert sein kann;
3. (C₃-C₈)-Cycloalkyl,
4. (C₃-C₆)-Cycloalkyl-(C₁-₃)-alkyl,
5. (C₆-C₁₂)-Aryl, vorzugsweise Phenyl,
6. (C₆-C₁₀)-Aryl-(C₁-C₄)-Alkyl,
7. (C₁-C₉)-Heteroaryl, welches teilweise oder vollständig hydriert sein kann,
8. (C₁-C₉)-Heteroaryl-(C₁-C₃)-alkyl, wobei der Heteroarylteil teilweise oder vollständig hydriert sein kann,
9. einen wie vorstehend unter 5., 6., 7. und 8. definierten Rest, substituiert mit 1 oder 2 gleichen oder verschiedenen Resten aus der Reihe Halogen, (C₁-C₄)-Alkyl, Hydroxy, Methoxy, Nitro, Cyano, CO₂R³, Trifluormethyl, -NR¹¹R¹² und 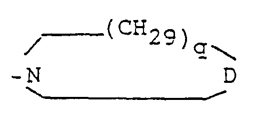
10. (C₁-C₆)-Alkyl, worin 1 bis alle H-Atome durch Fluor substituiert sind,
11. (C₂-C₆)-Alkenyl oder (C₃-C₆)-Alkenoyl,
12. (C₃-C₈)-Cycloalkenyl,
13. (C₃-C₈)-Cycloalkenyl-(C₁-C₃)-Alkyl,
14. (C₆-C₁₀)-Aryl-(C₁-C₄)-Alkyl,
15. (C₆-C₁₀)-Aryl-(C₃-C₆)-Alkenyl,
16. (C₁-C₉)-Hetaryl-(C₃-C₆)-Alkenyl,
17. (C₃-C₆)-Alkinyl,
18. (C₆-C₁₀)-Aryl-(C₃-C₆)-Alkinyl,
19. (C₁-C₉)-Hetaryl-(C₃-C₆)-Alkinyl,
20. R⁶, R⁹ gemeinsam mit dem sie tragenden N-Atom ein Hetaryl, das auch teilweise oder vollständig hydriert sein kann,
R⁷ Wasserstoff
R⁸ Wasserstoff oder -OR⁶
R¹¹,R¹² unabhängig voneinander Wasserstoff oder (C₁-C₄)-Alkyl
D -NR¹³, -O oder -CH₂
R¹³ Wasserstoff oder (C₁-C₄)-Alkyl
A Biphenylrest der mit einem Rest R¹⁵ oder mit R¹⁴ und R¹⁵ zusammen substituiert ist
R¹⁵ -SO₂-NR⁷-CO-NR⁶R⁹, -SO₂-NH-COO-R⁶, -SO₂-NH-SO₂-NR⁶R⁹, -SO₂-NH-CO-R⁶ oder -SO₂-NH-SO₂-R⁶; oder
R¹⁴ und R¹⁵ zusammen -CO-NH-SO₂- sein kann
R¹⁹
1. (C₁-C₆)-Alkyl
2. (C₃-C₈)-Cycloalkyl
3. Phenyl
4. Benzyl oder
5. den unter 1 definierten Rest, worin ein bis alle H-Atome durch Fluor substituiert sind
L -CH₂-
q Null und
r Null , 1 oder 2,
sowie deren physiologisch verträgliche Salze.
dadurch gekennzeichnet, daß man eine Verbindung der Formel (II) 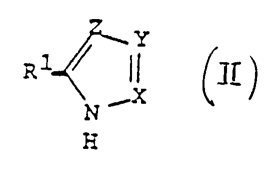 worin R¹, X, Y und Z wie oben definiert sind, alkyliert mit Verbindungen der Formel (III),
U-L-(O)_{q}-A (III)
worin L, A und q wie oben definiert sind, und U für eine Fluchtgruppe steht, gegebenenfalls temporär eingeführte Schutzgruppen wieder abspaltet, erhaltene Sulfonamide der Formel I gegebenenfalls in Urethane der Formel I überführt, erhaltene Sulfonamide der Formel I oder erhaltene Urethane der Formel I gegebenenfalls in Sulfonylharnstoffe der Formel I überführt und die erhaltenen Verbindungen der Formel (I) gegebenenfalls in ihre physiologisch verträglichen Salze überführt.

2. Verfahren zur Herstellung einer Verbindung der Formel I gemäß Anspruch 1, dadurch gekennzeichnet, daß die Verbindung Ethyl 1-[2'-n-Propylaminocarbonylaminosulfonylbiphenyl-4-yl) Methyl]-2-n-Butyl-4-Methylthiomidazol-5-Cärboxylat ist oder dessen physiologisch verträglichen Salze.

3. Verfahren zur Herstellung einer Verbindung der Formel I gemäß Anspruch 1, dadurch gekennzeichnet, daß die Verbindung 1-[2'-n-Propylaminocarbonylaminosulfonylbiphenyl-4yl)Methyl]-2-n-Butyl-4-Methylthioimidazol-5-Carbonsäure ist oder dessen physiologisch verträglichen Salze.

4. Verfahren zur Herstellung eines pharmazeutischen Präparates, dadurch gekennzeichnet, daß man eine Verbindung der Formel (I) oder ein Physiologisch verträgliches Salz einer solchen Verbindung erhältlich nach dem Verfahren gemäß Anspruch 1 zusammen mit einem physiologisch unbedenklichen Träger und gegebenemfalls weiteren Zusatz- oder Hilfsstoffen in eine geeignete Darreichungsform bringt.

## Claims (Claims for the following Contracting State(s): AT, BE, CH, DE, DK, FR, GB, IT, LI, LU, NL, PT, SE)

1. A compound of the formula (I) 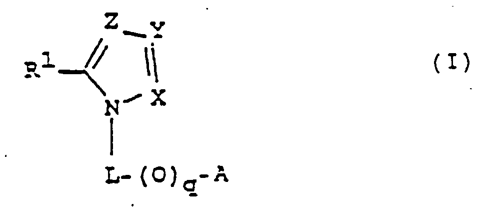 in which the symbols carry the following meanings:
Z is nitrogen
X, Y independently of one another are CR²
R¹ is (C₂-C₇)-alkyl, (C₃-C₇)-alkenyl or (C₃-C₇)-alkynyl,
R² is hydrogen, halogen, nitro, (C₁-C₃)-perfluoroalkyl, cyano, (C₁-C₁₀)-alkyl, (C₃-C₁₀)-alkenyl, -CH₂OR⁵, -S(O)ᵣ-R¹⁹, -CO-R⁸ or -O-R⁶,
R³ is
1. hydrogen,
2. (C₁-C₈)-alkyl,
3. (C₃-C₈)-cycloalkyl,
4. phenyl,
5. benzyl or
6. the radical defined under 2., in which 1 to all H atoms are substituted by fluorine
R⁵ is hydrogen or (C₁-C₆)-alkyl
R⁶, R⁹ are
1. hydrogen,
2. (C₁-C₆)-alkyl which can be substituted by 1-3 radicals from the group consisting of (C₂-C₁₀)-alkenyl, hydroxyl, amino, mono-(C₁-C₆)-alkylamino, di-(C₁-C₆)-alkylamino, (C₁-C₆)-alkoxycarbonylamino, (C₆-C₁₂)-aryl-(C₁-C₄)-alkoxycarbonylamino; (C₆-C₁₀)-aryl, (C₆-C₁₀) aryl-(C₁-C₃) -alkyl, (C₁-C₉)-heteroaryl, carboxyl and (C₁-C₄)-alkoxy-carbonyl, and also (C₁-C₆)-alkoxy which for its part can be substituted by 1-3 radicals from the group consisting of hydroxyl, (C₁-C₆)-alkoxy, amino, mono-(C₁-C₆)-alkylamino or di-(C₁-C₆)-alkyl-amino;
3. (C₃-C₈)-cycloalkyl,
4. (C₃-C₆)-cycloalkyl-(C₁₋₃)-alkyl,
5. (C₆-C₁₂)-aryl, preferably phenyl,
6. (C₆-C₁₀)-aryl-(C₁-C₄)-alkyl,
7. (C₁-C₉)-heteroaryl which can be partly or completely hydrogenated,
8. (C₁-C₉)-heteroaryl-(C₁-C₃)-alkyl,where the heteroaryl moiety can be partly or completely hydrogenated,
9. a radical as defined above under 5., 6., 7. and 8., substituted by 1 or 2 identical or different radicals from the group consisting of halogen, (C₁-C₄)-alkyl, hydroxyl, methoxy, nitro, cyano, CO₂R³, trifluoromethyl, -NR¹¹R¹² and 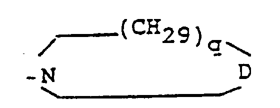
10. (C₁-C₆)-alkyl, in which 1 to all H atoms are substituted by fluorine,
11. (C₂-C₆)-alkenyl or (C₃-C₆)-alkenoyl,
12. (C₃-C₈)-cycloalkenyl,
13. (C₃-C₈)-cycloalkenyl-(C₁-C₃)-alkyl,
14. (C₆-C₁₀)-aryl-(C₁-C₄)-alkyl,
15. (C₆-C₁₀)-aryl-(C₃-C₆)-alkenyl,
16. (C₁-C₉)-hetaryl-(C₃-C₆)-alkenyl,
17. (C₃-C₆)-alkynyl,
18. (C₆-C₁₀)-aryl-(C₃-C₆)-alkynyl,
19. (C₁-C₉)-hetaryl-(C₃-C₆)-alkynyl,
20. R⁶, R⁹ together with the N atom carrying them are a hetaryl which can also be partly or completely hydrogenated,
R⁷ is hydrogen
R⁸ is hydrogen or -OR⁶
R¹¹,R¹² independently of one another are hydrogen or (C₁-C₄)-alkyl
D is -NR¹³, -O or -CH₂
R¹³ is hydrogen or (C₁-C₄)-alkyl
A is a biphenyl radical which is substituted by a radical R¹⁵ or by R¹⁴ and R¹⁵ together
R¹⁵ is -SO₂-NR⁷-CO-NR⁶R⁹, -SO₂-NH-COO-R⁶, -SO₂-NH- SO₂-NR⁶R⁹, -SO₂-NH-CO-R⁶ or -SO₂-NH-SO₂-R⁶; or
R¹⁴ and R¹⁵ together can be -CO-NH-SO₂-
R¹⁹ is
1. (C₁-C₆)-alkyl
2. (C₃-C₈)-cycloalkyl
3. phenyl
4. benzyl or
5. the radical defined under 1., in which one to all H atoms are substituted by fluorine
L is -CH₂-
q is zero and
r is zero, 1 or 2,
and their physiologically tolerable salts.

2. The compound of the formula I as claimed in claim 1, wherein the compound is ethyl 1-[2'-n-propylaminocarbonylaminosulfonylbiphenyl-4-yl)methyl]-2-n-butyl-4-methylthioimidazole-5-carboxylate or its physiologically tolerable salts.

3. The compound of the formula I as claimed in claim 1, wherein the compound is 1-[2'-n-propylaminocarbonylaminosulfonylbiphenyl-4-yl)methyl]-2-n-butyl-4-methylthioimidazole-5-carboxylate or its physiologically tolerable salts.

4. A process for the preparation of a compound of the formula (I) as claimed in one of claims 1 to 3, and of its physiologically tolerable salts, which comprises alkylating a compound of the formula (II) 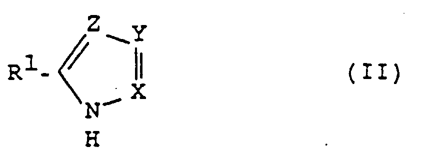 in which R¹, X, Y and Z are as defined in claim 1, using compounds of the formula (III)
**U-L-(O)**_{**q**}**-A** **(III)**
in which L, A and q are as defined in claim 1, and U is a leaving group, if appropriate removing temporarily introduced protective groups, if appropriate converting sulfonamides of the formula I obtained into urethanes of the formula I, if appropriate converting sulfonamides of the formula I obtained or urethanes of the formula I obtained into sulfonylureas of the formula I, and if appropriate converting the compounds of the formula (I) obtained into their physiologically tolerable salts.

5. The compound as claimed in one of claims 1 to 3 for use as a therapeutic.

6. The compound as claimed in one of claims 1 to 3 for use as a therapeutic for the treatment of high blood pressure.

7. A pharmaceutical preparation comprising a compound as claimed in one of claims 1 to 3.

8. A process for the production of a preparation as claimed in claim 7, which comprises bringing a compound of the formula (I) as claimed in claim 1 into a suitable administration form together with a physiologically acceptable excipient and, if appropriate, further additives or auxiliaries.

## Claims (Claims for the following Contracting State(s): ES, GR)

1. A process for the preparation of compounds of the formula (I) 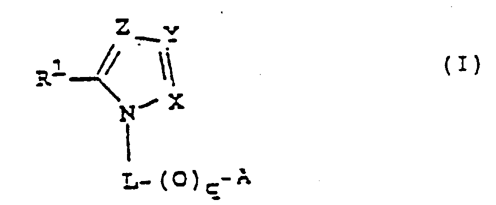 in which the symbols carry the following meanings:
Z is nitrogen
X, Y independently of one another are CR²
R¹ is (C₂-C₇)-alkyl, (C₃-C₇)-alkenyl or (C₃-C₇)-alkynyl,
R² is hydrogen, halogen, nitro, (C₁-C₃)-perfluoroalkyl, cyano, (C₁-C₁₀)-alkyl, (C₃-C₁₀)-alkenyl, -CH₂OR⁵, -S(O)ᵣ-R¹⁹, -CO-R⁸ or -O-R⁶,
R³ is
1. hydrogen,
2. (C₁-C₈)-alkyl,
3. (C₃-C₈)-cycloalkyl,
4. phenyl,
5. benzyl or
6. the radical defined under 2., in which 1 to all H atoms are substituted by fluorine;
R⁵ is hydrogen or (C₁-C₆)-alkyl
R⁶, R⁹ are
1. hydrogen,
2. (C₁-C₆)-alkyl which can be substituted by 1-3 radicals from the group consisting of (C₂-C₁₀)-alkenyl, hydroxyl, amino, mono-(C₁-C₆)-alkylamino, di-(C₁-C₆)-alkylamino, (C₁-C₆)-alkoxycarbonylamino, (C₆-C₁₂)-aryl-(C₁-C₄)-alkoxycarbonylamino; (C₆-C₁₀)-aryl, (C₆-C₁₀)aryl-(C₁-C₃)-alkyl, (C₁-C₉)heteroaryl, carboxyl and (C₁-C₄)-alkoxycarbonyl, and also (C₁-C₆)-alkoxy which for its part can be substituted by 1-3 radicals from the group consisting of hydroxyl, (C₁-C₆)-alkoxy, amino, mono-(C₁-C₆)-alkylamino or di-(C₁-C₆)-alkyl-amino;
3. (C₃-C₈)-cycloalkyl,
4. (C₃-C₆)-cycloalkyl-(C₁₋₃)-alkyl,
5. (C₆-C₁₂)-aryl, preferably phenyl,
6. (C₆-C₁₀)-aryl-(C₁-C₄)-alkyl,
7. (C₁-C₉)-heteroaryl which can be partly or completely hydrogenated,
8. (C₁-C₉)-heteroaryl-(C₁-C₃)-alkyl,where the heteroaryl moiety can be partly or completely hydrogenated,
9. a radical as defined above under 5., 6., 7. and 8., substituted by 1 or 2 identical or different radicals from the group consisting of halogen, (C₁-C₄)-alkyl, hydroxyl, methoxy, nitro, cyano, CO₂R³, trifluoromethyl, -NR¹¹R¹² and 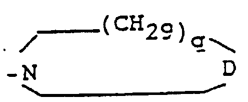
10. (C₁-C₆)-alkyl, in which 1 to all H atoms are substituted by fluorine,
11. (C₂-C₆)-alkenyl or (C₃-C₆)-alkenoyl,
12. (C₃-C₈)-cycloalkenyl,
13. (C₃-C₈)-cycloalkenyl-(C₁-C₃)-alkyl,
14. (C₆-C₁₀)-aryl-(C₁-C₄)-alkyl,
15. (C₆-C₁₀)-aryl-(C₃-C₆)-alkenyl,
16. (C₁-C₉)-hetaryl-(C₃-C₆)-alkenyl,
17. (C₃-C₆)-alkynyl,
18. (C₆-C₁₀)-aryl-(C₃-C₆)-alkynyl,
19. (C₁-C₉)-hetaryl-(C₃-C₆)-alkynyl,
20. R⁶, R⁹ together with the N atom carrying them are a hetaryl which can also be partly or completely hydrogenated,
R⁷ is hydrogen
R⁸ is hydrogen or -OR⁶
R¹¹,R¹² independently of one another are hydrogen or (C₁-C₄)-alkyl
D is -NR¹³, -O or -CH₂
R¹³ is hydrogen or (C₁-C₄)-alkyl
A is a biphenyl radical which is substituted by a radical R¹⁵ or by R¹⁴ and R¹⁵ together
R¹⁵ is -SO₂-NR⁷-CO-NR⁶R⁹, -SO₂-NH-COO-R⁶, -SO₂-NH- SO₂-NR⁶R⁹, -SO₂-NH-CO-R⁶ or -SO₂-NH-SO₂-R⁶; or
R¹⁴ and R¹⁵ together can be -CO-NH-SO₂-
R¹⁹ is
1. (C₁-C₆)-alkyl
2. (C₃-C₈)-cycloalkyl
3. phenyl
4. benzyl or
5. the radical defined under 1., in which one to all H atoms are substituted by fluorine
L is -CH₂-
q is zero and
r is zero, 1 or 2,
and their physiologically tolerable salts,
which comprises alkylating a compound of the formula (II) 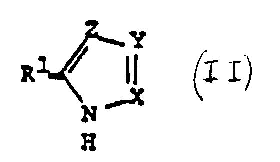 in which R¹, X, Y and Z are as defined above, using compounds of the formula (III)
**U-L-(O)**_{**q**}**-A** **(III)**
in which L, A and q are as defined above, and U is a leaving group, if appropriate removing temporarily introduced protective groups, if appropriate converting sulfonamides of the formula I obtained into urethanes of the formula I, if appropriate converting sulfonamides of the formula I obtained or urethanes of the formula I obtained into sulfonylureas of the formula I, and if appropriate converting the compounds of the formula (I) obtained into their physiologically tolerable salts.

2. Process for the preparation of a compound of the formula I as claimed in claim 1, wherein the compound is ethyl 1-[2'-n-propylaminocarbonylaminosulfonylbiphenyl-4-yl)methyl]-2-n-butyl-4-methylthioimidazole-5-carboxylate or its physiologically tolerable salts.

3. Process for the preparation of a compound of the formula I as claimed in claim 1, wherein the compound is 1- [2'-n-propylaminocarbonylaminosulfonylbiphenyl-4-yl)methyl]-2-n-butyl-4-methylthioimidazole-5-carboxylate or its physiologically tolerable salts.

4. A process for the production of a pharmaceutical preparation, which comprises bringing a compound of the formula (I) or a physiologically tolerable salt of such a compound obtainable by the process as claimed in claim 1 into a suitable administration form together with a physiologically acceptable excipient and, if appropriate, further additives or auxiliaries.

## Revendications (Revendications pour l'(les) Etat(s) contractant(s) suivant(s): AT, BE, CH, DE, DK, FR, GB, IT, LI, LU, NL, PT, SE)

1. Composé de formule (I), 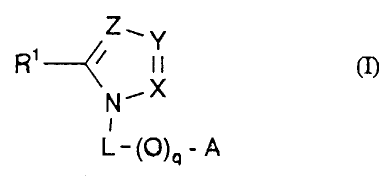 dans laquelle les symboles ont les significations suivantes :
Z un atome d'azote
X, Y indépendamment l'un de l'autre, CR²
R¹ un groupe alkyle (C₂-C₇), alcényle (C₃-C₇) ou alcynyle (C₃-C₇),
R² un atome d'hydrogène, un atome d'halogène, un groupe nitro, perfluoroalkyle (C₁-C₃), cyano, alkyle (C₁-C₁₀), alcényle (C₃-C₁₀), -CH₂OR⁵, S(O)ᵣ-R¹⁹ -CO-R⁸ ou -O-R⁶,
R³
1) un atome d'hydrogène,
2) un groupe alkyle (C₁-C₈),
3) un groupe cycloalkyle (C₃-C₈),
4) un groupe phényle,
5) un groupe benzyle ou
6) un radical défini en 2) dans lequel un à tous les atomes d'hydrogène sont substitués par un atome de fluor
R⁵ un atome d'hydrogène ou un groupe alkyle (C₁-C₆)
R⁶, R⁹
1) un atome d'hydrogène,
2) un groupe alkyle (C₁-C₆) qui peut être substitué par 1 à 3 radicaux choisis parmi un groupe alcoxy (C₁-C₆)qui peut être lui-même substitué par 1 à 3 restes choisis parmi les groupes hydroxy, alcoxy (C₁-C₆), amino, monoalkyl-(C₁-C₆)-amino, dialkyl-(C₁-C₆)-amino ; un groupe alcényle (C₂-C₁₀), hydroxy, amino, monoalkyl-(C₁-C₆)-amino, dialkyl-(C₁-C₆)-amino, alcoxy-(C₁-C₆)-carbonylamino, aryl-(C₆-C₁₂)-alcoxy-(C₁-C₄)-carbonylamino ; aryle (C₆-C₁₀), aryl-(C₆-C₁₀)-alkyle (C₁-C₃), hétéroaryle (C₁-C₉), carboxy et alcoxy-(C₁-C₄)-carbonyle ;
3) un groupe cycloalkyle (C₃-C₈),
4) un groupe cycloalkyl-(C₃-C₈)-alkyle (C₁-C₃),
5) un groupe aryle (C₆-C₁₂), de préférence phényle,
6) un groupe aryl-(C₆-C₁₀)-alkyle (C₁-C₄),
7) un groupe hétéroaryle (C₁-C₉)qui peut être partiellement ou totalement hydrogéné,
8) un groupe hétéroaryl-(C₁-C₉)-alkyle (C₁-C₃), la partie hétéroaryle pouvant être partiellement ou totalement hydrogénée,
9) un radical défini comme en 5), 6), 7) et 8), substitué par 1 ou 2 radicaux identiques ou différents choisis parmi un atome d'halogène, un groupe alkyle (C₁-C₄), hydroxy, méthoxy, nitro, cyano, CO₂R³, trifluorométhyl, -NR¹¹R¹² et 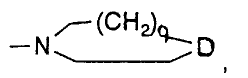
10) un groupe alkyle (C₁-C₆) dans lequel 1 à tous les atomes d'hydrogène sont substitués par un atome de fluor,
11) un groupe alcényle (C₂-C₆) ou alcénoyle (C₃-C₆),
12) un groupe cycloalcényle (C₃-C₈),
13) un groupe cycloalcényl-(C₃-C₈)-alkyle (C₁-C₃),
14) un groupe aryl-(C₆-C₁₀)-alkyle (C₁-C₄),
15) un groupe aryl-(C₆-C₁₀)-alcényle (C₃-C₆),
16) un groupe hétaryl-(C₁-C₉)-alcényle (C₃-C₆),
17) un groupe alcynyle (C₃-C₆),
18) un groupe aryl-(C₆-C₁₀)-alcynyle (C₃-C₆),
19) un groupe hétaryl-(C₁-C₉)-alcynyle (C₃-C₆),
20) R⁶, R⁹ forment ensemble avec l'atome d'azote qui les portent un groupe hétaryle qui peut être partiellement ou totalement hydrogéné,
R⁷ un atome d'hydrogène
R⁸ un atome d'hydrogène ou -OR⁶
R¹¹, R¹² indépendamment l'un de l'autre, représentent un atome d'hydrogène ou un groupe alkyle (C₁-C₄)
D -NR¹³, -O ou -CH₂
R¹³ un atome d'hydrogène ou un groupe alkyle (C₁-C₄)
A un radical biphényle substitué par un radical R¹⁵ ou par R¹⁴ et R¹⁵ ensemble
R¹⁵ -SO²-NR⁷-CO-NR⁶R⁹, -SO₂-NH-COO -R⁶, -SO₂-NH-SO₂-NR⁶R⁹, -SO₂-NH-CO-R⁶ ou -SO₂-NH-SO₂-R⁶ ; ou
R¹⁴ et R¹⁵ ensemble peuvent former -CO-NH-SO₂-
R¹⁹
1) un groupe alkyle (C₁-C₆),
2) un groupe cycloalkyle (C₃-C₈),
3) un groupe phényle,
4) benzyle ou
5) le reste défini en 1) dans lequel un à tous les atomes d'hydrogène sont substitués par un atome de fluor
L -CH₂-
q zéro et
r 0, 1 ou 2,
ainsi que ses sels physiologiquement acceptables.

2. Composé de formule (I) selon la revendication 1, caractérisé en ce que le composé est le 1-[2'-*n*-propylaminocarbonylaminosulfonylbiphényl-4-yl)méthyl]-2-n-butyl-4-méthylthioimidazole-5-carboxylate d'éthyle ou ses sels physiologiquement acceptables.

3. Composé de formule (I) selon la revendication 1, caractérisé en ce que le composé est l'acide 1-[2'-*n*-propylaminocarbonylaminosulfonylbiphényl-4-yl)méthyl]-2-n-butyl-4-méthylthioimidazole-5-carboxylique ou ses sels physiologiquement acceptables.

4. Procédé de préparation d'un composé de formule (I) selon une des revendications 1 à 3 ainsi que de ses sels physiologiquement acceptables, caractérisé en ce que l'on alkyle un composé de formule (Il) 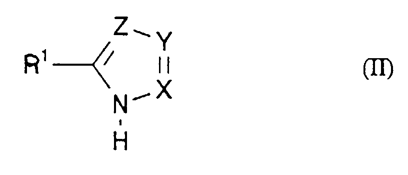 dans laquelle R¹, X, Y et Z sont définis comme à la revendication 1, avec des composés de formule (III)
U-L-(O)_{q}-A (III)
dans laquelle L, A et q sont définis comme à la revendication 1 et U représente un groupe partant, on élimine éventuellement les groupes protecteurs introduits temporairement, on transforme éventuellement les sulfonamides de formule I en uréthannes de formule I, on transforme éventuellement les sulfonamides de formule I ou les uréthannes de formule I obtenus en sulfonylurées de formule I, et on transforme éventuellement les composés de formule (I) obtenus en leurs sels physiologiquement acceptables.

5. Composé selon une des revendications 1 à 3 pour son utilisation comme médicament.

6. Composé selon une des revendications 1 à 3 pour son utilisation comme médicament pour le traitement de l'hypertension artérielle.

7. Préparation pharmaceutique contenant un composé selon une des revendications 1 à 3.

8. Procédé de fabrication d'une préparation selon la revendication 7, caractérisé en ce qu'un composé de formule (I) selon la revendication 1 est mis sous une forme de présentation appropriée avec un véhicule physiologiquement acceptable et éventuellement d'autres additifs ou adjuvants.

## Revendications (Revendications pour l'(les) Etat(s) contractant(s) suivant(s): ES, GR)

1. Procédé de préparation de composés de formule (I) 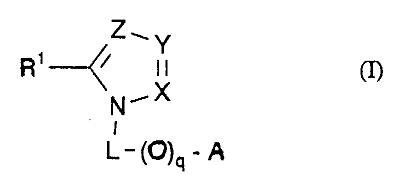 dans laquelle les symboles ont les significations suivantes :
Z un atome d'azote
X, Y indépendamment l'un de l'autre, CR²
R¹ un groupe alkyle (C₂-C₇), alcényle (C₃-C₇) ou alcynyle (C₃-C₇),
R² un atome d'hydrogène, un atome d'halogène, un groupe nitro, perfluoroalkyle (C₁-C₃), cyano, alkyle (C₁-C₁₀), alcényle (C₃-C₁₀), -CH₂OR⁵, S(O)ᵣ-R¹⁹ -CO-R⁸ ou -O-R⁶,
R³
1) un atome d'hydrogène,
2) un groupe alkyle (C₁-C₈),
3) un groupe cycloalkyle (C₃-C₈),
4) un groupe phényle,
5) un groupe benzyle ou
6) un radical défini en 2) dans lequel un à tous les atomes d'hydrogène sont substitués par un atome de fluor
R⁵ un atome d'hydrogène ou un groupe alkyle (C₁-C₆)
R⁶, R⁹
1) un atome d'hydrogène,
2) un groupe alkyle (C₁-C₆) qui peut être substitué par 1 à 3 radicaux choisis parmi un groupe alcoxy (C₁-C₆)qui peut être lui-même substitué par 1 à 3 restes choisis parmi les groupes hydroxy, alcoxy (C₁-C₆), amino, monoalkyl-(C₁-C₆)-amino, dialkyl-(C₁-C₆)-amino ; un groupe alcényle (C₂-C₁₀), hydroxy, amino, monoalkyl-(C₁-C₆)-amino, dialkyl-(C₁-C₆)-amino, alcoxy-(C₁-C₆)-carbonylamino, aryl-(C₆-C₁₂)-alcoxy-(C₁-C₄)-carbonylamino ; aryle (C₆-C₁₀), aryl-(C₆-C₁₀)-alkyle (C₁-C₃), hétéroaryle (C₁-C₉), carboxy et alcoxy-(C₁-C₄)-carbonyle ;
3) un groupe cycloalkyle (C₃-C₈),
4) un groupe cycloalkyl-(C₃-C₈)-alkyle (C₁-C₃),
5) un groupe aryle (C₆-C₁₂), de préférence phényle,
6) un groupe aryl-(C₆-C₁₀)-alkyle (C₁-C₄),
7) un groupe hétéroaryle (C₁-C₉)qui peut être partiellement ou totalement hydrogéné,
8) un groupe hétéroaryl-(C₁-C₉)-alkyle (C₁-C₃), la partie hétéroaryle pouvant être partiellement ou totalement hydrogénée,
9) un radical défini comme en 5), 6), 7) et 8), substitué par 1 ou 2 radicaux identiques ou différents choisis parmi un atome d'halogène, un groupe alkyle (C₁-C₄), hydroxy, méthoxy, nitro, cyano, CO₂R³, trifluorométhyl, -NR¹¹R¹² et 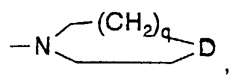
10) un groupe alkyle (C₁-C₆) dans lequel 1 à tous les atomes d'hydrogène sont substitués par un atome de fluor,
11) un groupe alcényle (C₂-C₆) ou alcénoyle (C₃-C₆),
12) un groupe cycloalcényle (C₃-C₈),
13) un groupe cycloalcényl-(C₃-C₈)-alkyle (C₁-C₃),
14) un groupe aryl-(C₆-C₁₀)-alkyle (C₁-C₄),
15) un groupe aryl-(C₆-C₁₀)-alcényle (C₃-C₆),
16) un groupe hétaryl-(C₁-C₉)-alcényle (C₃-C₆),
17) un groupe alcynyle (C₃-C₆),
18) un groupe aryl-(C₆-C₁₀)-alcynyle (C₃-C₆),
19) un groupe hétaryl-(C₁-C₉)-alcynyle (C₃-C₆),
20) R¹, R⁹ forment ensemble avec l'atome d'azote qui les portent un groupe hétaryle qui peut être partiellement ou totalement hydrogéné,
R⁷ un atome d'hydrogène
R⁸ un atome d'hydrogène ou -OR⁶
R¹¹, R¹² indépendamment l'un de l'autre, représentent un atome d'hydrogène ou un groupe alkyle (C₁-C₄)
D -NR¹³, -O ou -CH₂
R¹³ un atome d'hydrogène ou un groupe alkyle (C₁-C₄)
A un radical biphényle substitué par un radical R¹⁵ ou par R¹⁴ et R¹⁵ ensemble
R¹⁵ -SO₂-NR⁷-CO-NR⁶R⁹, -SO₂-NH-COO-R⁶, -SO₂-NH-SO₂-NR⁶R⁹, -SO₂-NH-CO-R⁶ ou -SO₂-NH-SO₂-R⁶ ; ou
R¹⁴ et R¹⁵ ensemble peuvent former -CO-NH-SO₂-
R¹⁹
1) un groupe alkyle (C₁-C₆),
2) un groupe cycloalkyle (C₃-C₈),
3) un groupe phényle,
4) benzyle ou
5) le reste défini en 1) dans lequel un à tous les atomes d'hydrogène sont substitués par un atome de fluor
L -CH₂-
q zéro et
r 0, 1 ou 2,
ainsi que ses sels physiologiquement acceptables,
caractérisé en ce que l'on alkyle un composé de formule (Il) 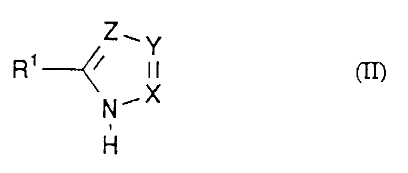 dans laquelle R¹, X, Y et Z sont définis comme ci-dessus, avec des composés de formule (III)
U-L-(O)_{q}-A (III)
dans laquelle L, A et q sont définis comme ci-dessus et U représente un groupe partant, on élimine éventuellement les groupes protecteurs introduits temporairement, on transforme éventuellement les sulfonamides de formule I en uréthannes de formule I, on transforme éventuellement les sulfonamides de formule I ou les uréthannes de formule I obtenus en sulfonylurées de formule I, et on transforme éventuellement les composés de formule (I) obtenus en leurs sels physiologiquement acceptables.

2. Procédé de préparation d'un composé de formule (I) selon la revendication 1, caractérisé en ce que le composé est le 1-[2'-*n*-propylaminocarbonylaminosulfonylbiphényl-4-yl)méthyl]-2-n-butyl-4-méthylthioimidazole-5-carboxylate d'éthyle ou ses sels physiologiquement acceptables.

3. Procédé de préparation d'un composé de formule (I) selon la revendication 1, caractérisé en ce que le composé est l'acide 1-[2'-*n*-propylaminocarbonylaminosulfonylbiphényl-4-yl)méthyl]-2-n-butyl-4-méthylthio-imidazole-5-carboxylique ou ses sels physiologiquement acceptables.

4. Procédé de fabrication d'une préparation pharmaceutique, caractérisé en ce que l'on met un composé de formule (I) ou un sel physiologiquement acceptable d'un tel composé obtenu selon le procédé de la revendication 1, sous une forme de présentation appropriée, avec un véhicule physiologiquement acceptable et éventuellement d'autres additifs ou adjuvants.
